# EUROPEAN PATENT APPLICATION

(11) **EP 2 105 133 A1**
(43) Date of publication of application: **30.09.2009**
(21) Application number: 08703030.0
(22) Date of filing: 10.01.2008
(51) Int. Cl.: A61K 31/198, A61K 47/10, A61K 47/12, A61K 47/14, A61K 47/26, A61K 47/32, A61K 47/34, A61K 47/36, A61K 47/38, A61K 47/42, A61P 25/16, A61P 43/00

(54) **INTRAGASTRIC FLOATING-TYPE LEVODOPA SUSTAINED-RELEASE PREPARATION**

(30) Priority: 15.01.2007 JP 2007005438
(71) Applicant: Kissei Pharmaceutical Co., Ltd., Matsumoto-shi Nagano 399-8710 (JP)
(72) Inventor: MOMOSE, Denichi, Matsumoto-shi Nagano 390-0221 (JP); ISSHIKI, Nobuyuki, Azumino-shi Nagano 399-8304 (JP); KAMADA, Noboru, Azumino-shi Nagano 399-8304 (JP)
(74) Representative: Hayes, Adrian Chetwynd
(86) International application number: PCT/JP2008/050159
(87) International publication number: WO 2008/087882

(57) **Abstract**

The present invention is to provide a levodopa preparation which is able to persistently release levodopa in the stomach, has a sufficient gastric residence time, is in an easily ingestible size and is able to be easily manufactured industrially with an object of maintaining a sustained concentration of levodopa in blood. It is a gastric retentive preparation for levodopa, **characterized in that**, the preparation contains a gastric resident layer showing a sufficient retentive property due to a high mechanical strength in the stomach and showing a good disintegrating property in an intestinal tract in addition to a drug releasing layer containing levodopa.

## Description

### TECHNICAL FIELD

The present invention relates to a gastric retention-type sustained-release levodopa preparation. More specifically, the invention relates to a gastric retention-type sustained-release levodopa preparation by which levodopa is persistently delivered to upper area of the small intestine which is an absorptive site for levodopa.

### BACKGROUND ART

Parkinson's disease is a chronic and progressive nerve disease showing ataxic symptoms including tremor, rigidity, dullness, postural reflex disturbance and gait disturbance. Although the causes therefor have not been clarified yet, it is likely that nigrostriatal dopamine-producing cells are fallen off in a gradually progressive manner.

Levodopa is a basic therapeutic agent for treating Parkinson's disease. Since levodopa is promptly metabolized by dopa decarboxylase, etc., it is sometimes necessary to ingest the dose of as high as 2 to 3 g and adverse actions such as nausea, vomiturition or palpitation are apt to happen.

Further, in patients suffering from Parkinson's disease, a wearing-off phenomenon or an on-off phenomenon where the effect of the drug often lowers and becomes gradually unstable often happens whereby it is necessary that levodopa is persistently administered.

In order to suppress the metabolism of levodopa so as to express the stable effect and to reduce the adverse action, a composite preparation comprising dopa and a dopa decarboxylase inhibitor which suppresses the dopa decarboxylation reaction and/or catechol-O-methyltransferase inhibitor which suppresses methylation of dopa has been developed.

Incidentally, in levodopa, its half-life in blood is as short as 60 to 90 minutes and it is mostly absorbed from the upper area of the small intestine whereby it is preferred to prepare a dosage form where levodopa is continuously delivered to the upper area of the small intestine. As to the preparation suitable for such an object, gastric retentive preparations of a mucosa-adhesion type, a unique-shape type, a swelling type and a floating type have been developed.

The gastric retentive preparation of a mucosa-adhesion type is intended to prolong the gastric residence time by inclusion therein of a mucosa-adherent substance that allows the preparation to adhere to the mucosa of the stomach. However, the preparation is unable to provide a sufficient gastric residence time because it does not readily adhere to the wall of a rapidly moving stomach and readily detaches itself from the mucosa by the metabolic turnover of the mucosa. There is also a problem of safety, such as a concern over the irritant effect of the preparation on the stomach mucosa.

The gastric retentive preparation of a unique-shape type is designed to elongate and extend at a particular temperature, pH or other conditions of the stomach environment so that it takes the unique shape of, for example, crossed rods or a windmill that permits the gastric retention. However, owing to the unique shape, the preparation is difficult to achieve and has the risk of damaging the stomach mucosa or preventing passage of food.

The gastric retentive preparation of a swelling type is intended to extend the gastric residence time with the use of an ingredient that swells upon contact with water and thereby increases the preparation to a size that makes passage through the pylorus difficult.

However, in the gastric retentive preparation of a swelling type, the attempt to prevent passage through the pylorus is made by increasing the size of preparation, which is achieved by the swelling of the preparation. Because of this, the preparation intrinsically lacks the mechanical strength necessary to resist the mechanical movement, such as contraction, of the stomach. Therefore, the preparation, when swollen, readily undergoes erosion and reduces its size. Further, because release of a drug is controlled by the erosion of the preparation, control of drug release is difficult when the preparation is designed to erode slowly, whereas, when designed to erode quickly the preparation reduces its size and is easily expelled from the stomach. For this reason, the gastric retentive preparation of a swelling type is greater in size than other types of gastric retentive preparations, which makes the preparation difficult to ingest.

Recently, a gastric retentive preparation of a swelling type has been reported in which the bilayer structure formed of a drug layer and a swelling layer containing an ingredient selected from xanthan gum, guar gum, hydroxypropyl methyl cellulose, polyvinyl alcohol and gum arabic allows the drug releasing property and the swelling property to be independently controlled (see Patent literature 1). However, assessment of the preparation by the inventors of the present invention revealed that the preparation still had the conventional problem; namely, the preparation has poor mechanical strength after swelling and readily undergoes erosion, and cannot provide a sufficient gastric residence time when it has an easily ingestible size, whereas ingestion is difficult when the preparation is designed to provide a sufficient gastric residence time.

There has been also a report of a preparation in which a portion of a polymer matrix that swells upon contact with the gastric fluid is surrounded by a band which is prepared from an insoluble substance such as polyethylene, polystyrene, ethylene-vinyl acetate copolymer or polycaprolactone that prevents the covered portion of the polymer matrix from swelling and thereby provides sufficient rigidity (mechanical strength) to withstand stomach contractions and delay expulsion of the preparation from the stomach (see Patent Publication 2). However, complicated procedures are required to surround a portion of the polymer matrix with the band, and preparation of such preparations on industrial scale is difficult to achieve.

The gastric retentive preparation of a floating type is intended to extend the gastric residence time by causing the - preparation to float in the stomach. However, because it takes time before the orally administered preparation floats, there are cases where the preparation is expelled from the stomach before it can float in the stomach.

To solve this problem, a gastric retentive preparation of a floating type has been proposed that has a bilayer structure including a layer formed of a pharmaceutical additive of a low bulk density (floating layer) and a drug-release control layer (see Patent literature 2). The floating layer of the preparation includes a low-density high-bulk cellulose derivative such as ethyl cellulose and provides a sufficient gastric residence time. However, since most of the floating layer is not eroded in the body, there is a problem that the digestive tract may be damaged, among others. Further, since the floating layer is excreted with the feces in its original form, it may give patients an uneasy feeling.

As to a sustained-release levodopa preparation or a combination preparation of levodopa with carbidopa, there is, for example, Sinemet CR (trade mark) sold in the United States and it releases levodopa and carbidopa during 4 to 6 hours by erosion of the polymer.

Patent literature 3 discloses a combination preparation of levodopa with carbidopa which is a controlled release orally administering prescription preparation containing hydroxypropyl cellulose which is a water-soluble polymer and vinyl acetate-crotonic acid copolymer which is less water-soluble polymer.

Patent literature 4 discloses a combination preparation of levodopa with carbidopa comprising a sustained release central core containing cellulose ether and microcrystalline cellulose and an outer layer having a releasing property of a medium degree.

Patent literature 5 discloses a gastric retentive combination preparation of levodopa with carbidopa of a swelling type containing hydrogel such as hydroxypropyl methyl cellulose or hydroxypropyl cellulose; an ultra-disintegrating agent selected from the group consisting of cross-linked carboxymethyl cellulose sodium, starch-sodium glycolate and cross-linked polyvinylpyrrolidone; and tannin.

Patent literature 6 discloses a gastric retentive combination preparation of levodopa with carbidopa of a floating type comprising a swelling membrane containing a binder selected from the group consisting of polyoxyethylene stearate, poloxamer, polyethylene glycol, glycerol palmitostearate, glyceryl monostearate, methyl cellulose and polyvinylpyrrolidone; a gas-generating agent selected from the group consisting of sodium bicarbonate, sodium carbonate, glycine-sodium carbonate, potassium carbonate, calcium carbonate, magnesium carbonate and a mixture thereof; and polyvinyl alcohol.

Nonpatent literature 1 discloses a gastric retentive preparation of levodopa in which a preparation in a sheet shape containing (1) hydrolyzed gelatin, L-polylactic acid and ethyl cellulose or (2) Eudragit L (trade name) and ethyl cellulose is folded and sealed into a capsule and, after administration, the folded sheet-shaped preparation opens in the stomach whereby the preparation is retained in the stomach.
Patent literature 1: JP-A-2005-132803
Patent literature 2: International Publication Pamphlet WO 99/07342
Patent literature 3: JP-T-06-67830 (the term "JP-T" as used herein means a published Japanese translation of a PCT patent application)
Patent literature 4: International Publication Pamphlet WO 2000/15197
Patent literature 5: International Publication Pamphlet WO 2002/000213
Patent literature 6: International Publication Pamphlet WO 2004/032906
Nonpatent literature 1: Klausner, E. A. , et al. Pharmaceutical Research, 2003, Vol. 20, No. 9, pages 1466 to 1473

### DISCLOSURE OF THE INVENTION

An object of the present invention is to provide a levodopa preparation in an easily ingestible size being able to release levodopa sustainedly in the stomach.

The inventors of the present invention diligently worked and found that, in the preparations in the same size, discharge from the stomach is better when the mechanical strength is lower and that the above problems are able to be solved by the use of a gastric retentive layer being unclassified into any of known categories that has sufficient retentive properties owing to a high mechanical strength in the stomach and has desirable disintegrating properties in the intestinal tract and accomplished the present invention.

Thus, the gist of the present invention lies in a gastric retentive preparation including a gastric resident layer and a drug releasing layer containing levodopa as a medicament, wherein the gastric resident layer contains a polymer ingredient which shows high solubility or swelling property in a weakly acidic or a weakly alkaline medium than in an acidic medium.

### ADVANTAGEOUS EFFECTS OF THE INVENTION

The gastric retentive preparation in accordance with the present invention retains in the stomach for long time and continues in releasing levodopa whereby it is able to well retain the concentration of levodopa in blood.

### BRIEF DESCRIPTION OF THE DRAWINGS

[Fig. 1] Fig.1 shows a diagram illustrating an apparatus used for strength test.
[Fig. 2] Fig.2 shows a diagram illustrating the changes in concentration of levodopa in blood, in which the vertical axis represents concentration and the horizontal axis represents time.
[Fig. 3] Fig.3 shows a diagram illustrating the changes in concentration of levodopa in blood, in which the vertical axis represents concentration and the horizontal axis represents time.
[Fig. 4] Fig.4 shows the dissolution rate of levodopa from the preparation of Example 7.
[Fig. 5] Fig.5 shows the dissolution rate of levodopa-carbidopa from the preparation of Example 1.
[Fig. 6] Fig.6 shows the dissolution rate of levodopa-carbidopa from the preparation of Example 3.
[Fig. 7] Fig.7 shows the dissolution rate of levodopa-carbidopa from the preparation of Example 4.
[Fig. 8] Fig.8 shows the dissolution rate of levodopa-carbidopa from the preparation of Example 5.
[Fig. 9] Fig.9 shows the dissolution rate of levodopa-carbidopa from the preparation of Example 6.
[Fig. 10] Fig.10 shows the dissolution rate of levodopa-carbidopa from the preparation of Example 8.
[Fig. 11] Fig.11 shows the dissolution rate of levodopa-carbidopa from the preparation of Example 9.
[Fig. 12] Fig.12 shows the dissolution rate of levodopa-carbidopa from the preparation of Example 10.
[Fig. 13] Fig.13 shows the dissolution rate of levodopa-carbidopa from the preparation of Example 11.
[Fig. 14] Fig.14 shows the dissolution rate of levodopa-carbidopa from the preparation of Example 12.
[Fig. 15] Fig.15 shows the dissolution rate of levodopa-carbidopa from the preparation of Example 13.
[Fig. 16] Fig.16 shows the dissolution rate of levodopa-carbidopa from the preparation of Example 14.
[Fig. 17] Fig.17 shows the dissolution rate of levodopa-carbidopa from the preparation of Example 15.
[Fig. 18] Fig.18 shows the dissolution rate of levodopa-carbidopa from the preparation of Example 16.
[Fig. 19] Fig.19 shows the dissolution rate of levodopa-carbidopa from the preparation of Example 17.
[Fig. 20] Fig.20 shows the dissolution rate of levodopa-carbidopa from the preparation of Example 18.
[Fig. 21] Fig.21 shows the dissolution rate of levodopa-carbidopa from the preparation of Example 19.
[Fig. 22] Fig.22 shows the dissolution rate of levodopa-carbidopa from the preparation of Example 20.
[Fig. 23] Fig.23 shows the dissolution rate of levodopa-carbidopa from the preparation of Example 21.

### BEST MODE FOR CARRYING OUT THE INVENTION

A gastric retentive preparation containing levodopa of the present invention comprises a gastric resident layer and a drug releasing layer, wherein the gastric resident layer does not disintegrate in the stomach and disintegrates in the intestinal tract.

As used herein, the term "does not disintegrate in the stomach" means that the gastric resident layer is not eroded by the gastric fluid or the contraction motion of the stomach and the shape thereof remains stable. The gastric retentive preparation having the gastric resident layer that does not disintegrate in the stomach is, for example, one in which the gastric resident layer has the shortest diameter (hereinafter referred to as "small diameter") of 7 mm or more, and more preferably 8 mm or more, which makes passages through the pylorus difficult, as measured after stirring the preparation in the first fluid at 200 rpm at 37°C for 15 hours under the conditions of the paddle method in the dissolution test in accordance with the Japanese Pharmacopoeia, Fourteenth Edition (hereinafter referred to as "JP").

Preferably, the preparation has mechanical strength against the mechanical action such as the contraction motion of the stomach, because the preparation is subjected to such action in the stomach in addition to the erosion by the gastric fluid. An example of a preparation with mechanical strength is one that can retain its shape in a test simulating the mechanical action of the stomach, or more specifically one that has a maximum load of 5000 g or more when compressed by 60 mm per minute to a thickness of 1 mm after being rotated at 200 rpm at 37°C for 10 hours in a horizontally laid 50 mL volume conical tube (30 × 115 mm) containing 50 g of glass beads having an outer diameter of about 4 mm to 5 mm and 30 mL of the JP first fluid, the JP first fluid being replaced every 2.5 hours during the rotation. A preparation with a maximum load below 5000 g is apt to be discharged from the stomach and readily undergoes erosion and, therefore, it may reduce to a size small enough to pass through the pylorus. It is therefore preferable that the preparation has a maximum load of 10000 g or more. The preparation after the test preferably has a small diameter of 7 mm or more, and more preferably 8 mm or more, which makes passage through the pylorus difficult.

The term "disintegrates in the intestinal tract" means that the gastric resident layer quickly disintegrates in the intestinal fluid. An example of a gastric resident layer that disintegrates in the intestinal tract is one that has a maximum diameter (hereinafter referred to as "large diameter") of 6 mm or less as measured after the preparation, taken out of the first fluid in which the preparation had been stirred under the conditions of the paddle method in the dissolution test in accordance with the JP was stirred in the second fluid at 200 rpm at 37°C for 9 hours under the same conditions. It can be said that the preparation has better disintegrating properties when the gastric resident layer has smaller large diameters after the test. It is therefore preferable that the preparation has a large diameter of 4 mm or less, and particularly 2 mm or less. A preparation with the gastric resident layer that completely disintegrates is most preferable.

The gastric resident layer of the gastric retentive preparation contains a polymer ingredient (hereinafter referred to as "pH-dependent polymer ingredient") which shows higher solubility or swellability in a weakly acidic to weakly alkaline medium such as intestinal fluid (pH 5 to 8) than in an acidic medium such as gastric fluid (pH 1 to 3) whereby it is now possible to make a preparation which does not disintegrate in the stomach but disintegrates in the intestinal tract.

The pH-dependent polymer ingredient may be an enteric ingredient and/or a polymer ingredient selected from the group consisting of carboxymethyl cellulose, polyacrylic acids and polysaccharides having a carboxyl group or a sulfo group (hereinafter such a polymer ingredient will be referred to as "acidic functional group-containing polymer ingredient").

Any enteric ingredient may be used so far as it is a substance commonly used for enteric preparations and that dissolves or swells in an aqueous solution at a pH of 5.5 or higher. For example, the enteric ingredient may be enteric cellulose derivatives such as cellulose acetate phthalate, cellulose acetate succinate, methyl cellulose phthalate, hydroxymethyl ethyl cellulose phthalate, hydroxypropyl methyl cellulose phthalate, hydroxypropyl methyl cellulose acetate succinate, cellulose acetate trimellitate or carboxymethyl ethyl cellulose; enteric acrylic acid copolymers such as a methyl methacrylate-methacrylic acid copolymer, a styrene-acrylic acid copolymer, a methyl acrylate-methacrylic acid-octyl acrylate copolymer, a methacrylic acid-ethyl acrylate copolymer (Eudragit L 100-55 and Eudragit L 30D-55, trade mark), a methyl acrylate-methyl methacrylate-methacrylic acid copolymer (Eudragit FS 30D, trade mark) or a methacrylic acid-methyl methacrylate copolymer (Eudragit L 100 and Eudragit S 100, trade mark); enteric polyvinyl derivatives such as polyvinyl butyrate phthalate or polyvinyl acetoacetal phthalate; and enteric maleic acid-vinyl copolymers such as a copolymer of vinyl acetate and maleic acid anhydride, a copolymer of vinyl butyl ether and maleic acid anhydride or a copolymer of styrene and maleic acid monoester. Among these, the enteric cellulose derivatives and enteric acrylic acid copolymers are preferable. The enteric ingredient may be used either individually or in a combination of two or more kinds with any proportions.

When an enteric ingredient is used as a pH-dependent polymer ingredient, the amount of the enteric ingredient in the gastric resident layer is usually not less than 50% by weight, preferably not less than 60% by weight, more preferably not less than 70% by weight and, still more preferably, not less than 80% by weight of the gastric resident layer.

Examples of carboxymethyl cellulose include carmellose, carmellose calcium, carmellose sodium, and croscarmellose sodium. Examples of polyacrylic acids include carboxyvinyl polymer and polycarbophil calcium. Examples of polysaccharides having a carboxyl group or a sulfo group include sodium carboxymethyl starch, alginic acid, xanthan gum, gellan gum, hyaluronic acid, carrageenan, chondroitin sulfate and dextran sulfate. Among these, the carboxymethyl celluloses and polyacrylic acids are preferable. The acidic functional group-containing polymer ingredient may be used either individually or in a combination of two or more kinds with any proportions, or in further combination with the enteric ingredient.

When an acidic functional group-containing polymer ingredient is used as a pH-dependent polymer ingredient, the amount of the acidic functional group-containing polymer ingredient in the gastric resident layer is usually 1 to 50% by weight, preferably 2 to 30% by weight and, more preferably, 3 to 20% by weight of the gastric resident layer.

When solubility or swellability of the pH-dependent polymer ingredient is high, there are cases where the gastric retentive preparation readily disintegrates. Such tendency is prominent when the acidic functional group-containing polymer ingredient is used as an ingredient. In this case, the gastric retentive preparation that retains its shape in the stomach and shows a disintegrating property in the intestinal tract can be realized by inclusion of a hydrophobic ingredient that exhibits a moderate strength in water. Examples of such a hydrophobic ingredient include: cellulose derivatives such as ethyl cellulose or cellulose acetate; acrylic acid copolymers such as an ethyl acrylate-methyl methacrylate copolymer (Eudragit NE; trade mark) or an ethyl acrylate-methyl methacrylate-trimethylammonioethyl methacrylate chloride copolymer (Eudragit RL and Eudragit RS; trade marks); polyvinyl derivatives polymers such as an ethylene-vinyl acetate copolymer or vinyl acetate resin; oils and fats such as hydrogenated oil, whale wax, bee wax, carnauba wax, lanolin or cacao buffer; higher fatty acids such as capric acid, lauric acid, myristic acid, palmitic acid, stearic acid, arachic acid or behenic acid; higher fatty acid esters such as higher fatty acid alkyl esters, polyglycerol higher fatty acid esters, higher fatty acid sorbitans, higher fatty acid polyethylene glycols or sucrose higher fatty acid esters; higher alcohols such as lauryl alcohol, myristyl alcohol, cetyl alcohol, stearyl alcohol or behenyl alcohol; hydrocarbons such as polyethylene, polystyrene, polyisobutylene or microcrystalline wax; polycaprolactone; polylactate; polyglycolate; and polyamides. Examples of higher fatty acid alkyl esters include myristyl myristate, cetyl palmitate, cholesteryl stearate and batyl monostearate. Examples of polyglycerol higher fatty acid esters include glyceryl myristate, glyceryl monostearate, glyceryl distearate, diglyceyrl monostearate, tetraglceryl monostearate, tetraglyceryl tristearate, tetraglceryl pentastearate, hexaglyceryl monostearate, hexaglyceryl tristearate, hexaglyceryl tetrabehenate, decaglyceryl monostearate, decaglyceryl distearate, decaglyceryl tristearate, decaglyceryl pentastearate, decaglyceryl pentahydroxystearate, decaglyceryl heptastearate and decaglyceryl decastearate. Examples of higher fatty acid sorbitans include sorbitan monopalmitate, sorbitan monostearate, sorbitan sesquistearate and sorbitan tristearate. Examples of higher fatty acid polyethylene glycols include ethylene glycol monostearate, polyethylene glycol monostearate, ethylene glycol distearate, diethylene glycol stearate and polyethylene glycol distearate. Examples of sucrose higher fatty acid esters include sucrose stearate ester, sucrose palmitate ester, sucrose oleate ester, sucrose laurate ester, sucrose behenate ester and sucrose erucate ester. Among these, the cellulose derivatives or oils and fats are preferable. The hydrophobic ingredient may be used either individually or in a combination of two or more kinds with any proportions.

The proportions of the pH-dependent polymer ingredient and the hydrophobic ingredient may be decided such that the residence time in human is 12 hours or more and, preferably, 12 to 24 hours depending upon the types of the ingredients used and that the gastric resident layer quickly disintegrates in the intestinal tract. The hydrophobic ingredient is generally in the range of 0 part by weight to 40 parts by weight, preferably 0 part by weight to 30 parts by weight and, particularly preferably, 0 part by weight to 20 parts by weight, with respect to 1 part by weight of the pH-dependent polymer ingredient in the gastric resident layer.

When an acidic functional group-containing polymer ingredient and hydrophobic ingredient are contained in the gastric resident layer, the proportions of the acidic functional group-containing polymer ingredient and the hydrophobic ingredient may be decided such that the residence time in human is 12 hours or longer or, preferably, 12 to 24 hours, depending on the types of the ingredients used and that the gastric resident layer quickly disintegrates in the intestinal tract. The hydrophobic ingredient is generally in the range of 1 to 40 part (s) by weight, preferably 2 to 30 parts by weight or, more preferably, 5 to 20 parts by weight with respect to 1 part by weight of the acidic functional group-containing polymer ingredient in the gastric resident layer.

By including a water-soluble ingredient in the gastric resident layer with the pH-dependent polymer ingredient and a necessary hydrophobic ingredient, the disintegrating property of the gastric resident layer can be controlled to adjust the gastric residence time and the ease of disintegration in the intestinal tract. Examples of a water-soluble ingredient include hydroxyalkyl celluloses such as hydroxypropyl cellulose, hydroxypropylmethyl cellulose (2208, 2906 and 2910) or hydroxyethyl cellulose; polyvinyl derivatives such as povidone, crospovidone or polyvinyl alcohol; polyethylene oxides; methyl cellulose; gelatin; polysaccharides such as pregelatinized starch, partially pregelatinized starch, pullulan, dextrin, sodium alginate or gum arabic; Macrogols such as Macrogol 400, Macrogol 1500, Macrogol 4000, Macrogol 6000 or Macrogol 20000; sugars such as lactose, saccharose, trehalose or glucose; and sugar alcohols such as mannitol, xylitol, sorbitol, erythritol or maltitol. Among these, hydroxyalkyl celluloses, polyvinyl derivatives, polyethylene oxides, sugars or sugar alcohols are preferred.

The proportion of the water-soluble ingredient may be appropriately decided such that the gastric residence time is 12 hours or longer or, preferably, 12 to 24 hours and that the gastric resident layer quickly disintegrates in the intestinal tract.

In the case where the gastric resident layer containing the enteric ingredient additionally contains the water-soluble ingredient, the gastric residence time may become insufficient due to lowering in the mechanical strength of the gastric resident layer when the proportion of the water-soluble ingredient in the gastric resident layer exceeds 50% by weight. For this reason, the proportion of the enteric ingredient in the gastric resident layer is preferably not less than 50% by weight, more preferably, not less than 60% by weight and, still more preferably, not less than 70% by weight of the gastric resident layer.

To be more specific, when an enteric ingredient and a water-soluble ingredient are contained in the gastric resident layer, the amounts of the enteric ingredient and the water-soluble ingredient in the gastric resident layer are that, preferably, the enteric ingredient is 50% by weight or more and the water-soluble ingredient is 50% by weight or less; more preferably, the enteric ingredient is 60 to 90% by weight and the water-soluble ingredient is 10 to 40% by weight; and, still more preferably, the enteric ingredient is 70 to 85% by weight and the water-soluble ingredient is 15 to 30% by weight of the gastric resident layer.

When the gastric resident layer containing the acidic functional group-containing polymer ingredient and the hydrophobic ingredient additionally contains the water-soluble ingredient, the gastric residence time may become insufficient due to lowering in the mechanical strength when the proportion of the water-soluble ingredient in the gastric resident layer exceeds 60% by weight. For this reason, it is preferable that the upper limit of the proportion of the water-soluble ingredient in the gastric resident layer be 60% by weight or less and, particularly, 50% by weight or less. On the other hand, the ease of disintegration in the intestinal tract may suffer when the proportion of the water-soluble ingredient in the gastric resident layer is below 5% by weight. It is therefore preferable that the lower limit of the proportion of the water-soluble ingredient in the gastric resident layer be 5% by weight or more and, particularly, 10% by weight or more of the gastric resident layer.

The gastric resident layer may additionally include an excipient, a binder, a fluidizer, a lubricant or the like. Examples of an excipient include microcrystalline cellulose, corn starch and anhydrous calcium hydrogen phosphate. Examples of a binder include methyl cellulose, hydroxypropyl cellulose, hydroxypropylmethyl cellulose, povidone, polyvinyl alcohol, pullulan, polyethylene glycol, gelatin, gum arabic, pregelatinized starch and partially pregelatinized starch. Examples of a fluidizer include light anhydrous silicic acid and hydrate silicon dioxide. Examples of a lubricant include magnesium stearate, calcium stearate, polyoxyl stearate, talc, sucrose fatty acid ester, dimethyl polysiloxane and sodium stearyl fumarate. The proportion of the ingredient above in the gastric resident layer may be appropriately decided according to the type and amount of the other ingredients used.

The gastric resident layer may be prepared by ordinary methods. For example, a method may be used in which the foregoing ingredients are mixed together and the admixture is compressed to form the layer under a pressure of 3 kN to 20 kN and, preferably, 5 kN to 15 kN.

A drug releasing layer may be designed, if necessary, to have an immediate-release drug releasing function together with a sustained-release drug releasing layer. For example, it may be made into a three-layered tablet comprising gastric resident layer, sustained-release drug releasing layer and immediate-release drug releasing layer or may be made into a preparation where a gastric resident layer and a sustained-release drug releasing layer are coated with an immediate-release drug releasing layer.

As a result of applying such an immediate-release drug releasing layer to the present preparation, it is now possible that the concentration of levodopa in blood is able to be made to reach the effective blood level immediately after the administration whereby, after the administration, quick improvement in the symptoms of Parkinson's disease is able to be achieved. Further, due to the dissolution of the drug from the sustained-release layer after that, it is possible to keep the constant blood level for long time.

Although levodopa contained in the sustained-release drug releasing layer and the immediate-release drug releasing layer may be made into any ratio, it is usual that the ratio of levodopa in the sustained-release drug releasing layer to the total weight of levodopa in the preparation is not less than 50%, preferably not less than 70% and, more preferably, not less than 80%.

As to the ingredient for the drug releasing layer, various kinds of ingredients which have been known for persons skilled in the art may be appropriately selected so as to give function(s) of immediately releasing property and/or sustainedly releasing property. Examples of the ingredient as such include microcrystalline cellulose; alkyl celluloses such as methyl cellulose or ethyl cellulose; hydroxyalkyl celluloses such as hydroxypropyl cellulose, low substituted hydroxypropyl cellulose, hydroxypropylmethyl cellulose (2208, 2906, 2910) or hydroxyethyl cellulose; carboxymethyl celluloses such as carboxymethyl cellulose, carboxymethyl cellulose calcium or croscarmellose sodium; gums such as guar gum, xanthan gum or gellan gum; polyethylene oxides; aminoalkyl methacrylate copolymers; acrylic acid copolymers; carboxyvinyl polymers; polyvinylpyrrolidones; polyvinyl alcohols; macrogols; oils and fats such as carnauba wax or hydrogenated oil; starches such as corn starch, potato starch, wheat starch, dextrin, pregelatinized starch, partially pregelatinized starch, carboxymethyl starch sodium or pullulan; sugars such as lactose or saccharose; sugar alcohols such as mannitol, xylitol, sorbitol or maltitol; minerals such as kaolin, talc, magnesium stearate, titanium oxide, precipitated calcium carbonate or calcium hydrogen phosphate; and plasticizers such as triethyl citrate, propylene glycol, triacetin or medium-chain fatty acid triglyceride. Desired sustained release conditions can be set by appropriately selecting these ingredients. Among these ingredients, it is preferable to include hydroxyalkyl cellulose or polyethylene oxide. The ingredient may be used either individually or in a combination of two or more kinds with any proportions.

Solubility of levodopa and carbidopa which will be mentioned later is high under an acidic condition and is low under a weakly acidic to basic condition. An acidic ingredient may be included in the drug releasing layer so that a preferred level in blood is able to be maintained even when gastric pH changes before and after the meal. As to the acidic ingredients, there may be used, for example, inorganic acid; organic acid such as fumaric acid, maleic acid, malonic acid, oxalic acid, ascorbic acid, malic acid, citric acid or amino acid; enteric ingredient; carboxymethyl celluloses; and polymer ingredient selected from the group consisting of polyacrylic acids and polysaccharides having carboxyl group or sulfo group. Each of those ingredients may be used solely or two or more thereof may be used jointly by mixing in any proportion. Among these, preferred ones are organic acid, carboxymethyl celluloses and polymer ingredient selected from the group consisting of polyacrylic acids and polysaccharides having carboxyl group or sulfo group. When an acidic ingredient is included, it is also possible to enhance the stability of levodopa and carbidopa in the drug releasing layer.

With regard to enteric ingredients, carboxymethyl celluloses, polyacrylic acids and polysaccharides having carboxyl group or sulfo group, there may be used the same ingredient which is used for a gastric resident layer.

In the drug releasing layer, it is preferred that a dopa decarboxylase inhibitor such as carbidopa or benserazide is contained therein together with levodopa. As a result thereof, it is possible that metabolism of levodopa in a peripheral site is inhibited, transfer of levodopa into the brain is enhanced and an adverse action by dopamine is reduced.

When a catechol-O-methyltransferase (COMT) inhibitor such as entacapone or tolcapone is contained as a medicament therein, it is preferred since levodopa is able to be transferred into the brain more efficiently.

The drug releasing layer may further contain a medicament having an effect of improving the parkinsonism and/or an effect of reducing the adverse action including, for example, anti-cholinergic agent such as trihexyphenidyl hydrochloride; dopamine receptor stimulator such as ergot or non-ergot alkaloid; MAO-B inhibitor such as selegiline hydrochloride; nor-epinephrine supplementing agent such as droxidopa; and dopamine release promoter such as amantadine hydrochloride.

Levodopa contained in the drug releasing layer of the gastric retentive preparation of the present invention is administered usually in the range of 20 mg to 2000 mg or, preferably, in the range of 100 mg to 800 mg per day for an adult. When at least one member selected from the dopa decarboxylase inhibitor and the COMT inhibitor is further contained in the drug releasing layer of the gastric retentive preparation of the present invention, the compounding ratio of those drugs may be appropriately selected depending upon age, sex, body weight and symptom of a patient and upon combination of the drugs. For example, when carbidopa is further contained in the drug releasing layer, carbidopa is administered usually in the range of 2 mg to 500 mg or, preferably, in the range of 20 mg to 200 mg per day for an adult. When a COMT inhibitor is further contained in the drug releasing layer, the COMT inhibitor is administered usually in the range of 20 mg to 2000 mg or, preferably, in the range of 100 mg to 800 mg per day for an adult.

The drug releasing layer may be prepared by an ordinary method. For example, a method may be used in which the foregoing ingredient and drug are mixed together and the admixture is compressed to form the layer under a pressure of 3 kN to 20 kN and, preferably, 5 kN to 15 kN.

A gastric retentive preparation according to the present invention may be prepared by methods commonly used for the preparation of bilayer or multilayer tablets. For example, the gastric retentive preparation may be prepared by a method in which the ingredients respectively used for the gastric resident layer and the drug releasing layer are separately loaded into a tableting machine and are compressed into a bilayer or multilayer tablet under a pressure of 3 kN to 20 kN or, preferably, 5 kN to 15 kN. The bonding of the gastric resident layer and the drug releasing layer may be made, for example, by tablet compression of the gastric resident layer and the drug releasing layer, or by tablet compression using a bonding layer between the gastric resident layer and the drug releasing layer, the bonding layer including a binder such as hydroxypropyl cellulose.

The gastric resident layer including the enteric ingredient, particularly an acrylic acid copolymer, is relatively hydrophobic while the drug releasing layer including hydroxyalkyl cellulose or polyethylene oxide is relatively hydrophilic. For this reason, desirable bonding may not be achieved by the simple tablet compression of the two materials. It is therefore preferable, when bonding is made, to include the water-soluble ingredient in the gastric resident layer and thereby improve the strength of bonding.

In the gastric retentive preparation of the present invention, although the proportion of the gastric resident layer to the drug releasing layer may be in any proportion, the proportion of the gastric resident layer to the total weight of the preparation is usually 5 to 50% by weight, preferably 10 to 45% by weight and, more preferably, 15 to 40% by eight.

The gastric retentive preparation may have any shape so far as it is suited for manufacture, ingestion or the like. Some of the possible shapes are in lens, disc, circle, caplet, ellipse, triangle, quadrangle, pentagon, hexagon, flower and oval.

Preferably, the gastric retentive preparation is sized so that the lower limit of its diameter (short axis in the case of irregular shape) is greater than 6 mm and, particularly, 7 mm or greater. A sufficient gastric residence time may not be obtained when the diameter is 6 mm or less. The upper limit of diameter (long axis in the case of irregular shape) may be any length provided that it permits ingestion. Generally, the upper limit of diameter is 30 mm or less.

For enhancing the identification, the gastric retentive preparation may contain a colorant, such as a dye, in the drug releasing layer and/or the gastric resident layer. Any colorant may be used so far as it is able to be used as a pharmaceutical and examples thereof include food dyes such as Food Yellow 5, Food Red 2 or Food Blue 2; food lake dyes; ferric oxide; yellow ferric oxide; yellow iron oxide; and black iron oxide.

### EXAMPLES

The following will describe the present invention in more detail based on Examples. The present invention is not limited in any ways by the following Examples.

### (Dissolution test)

According to the paddle method described in the JP dissolution testing method, a test preparation was placed in a sinker and was immersed in the JP first fluid (900 mL), which was then stirred for 15 hours at 37°C at a paddle speed of 200 rpm. The test preparation was gently taken out and was immersed in the JP second fluid (900 mL). After stirring the solution for 9 hours at 37°C at a paddle speed of 200 rpm, the large diameter of the gastric resident layer of the preparation was measured with a caliper. In the case where no remains of the gastric resident layer were observed, or when the remains of the gastric resident layer appeared spongy or only a small portion of the soft material was present, the time from the start of stirring was recorded as the dissolution time.

### (Strength test)

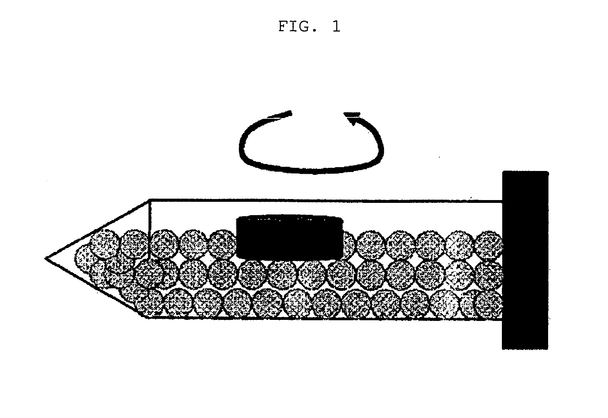

A 50 mL volume Falcon^{™} conical tube (30 × 115 mm) for centrifugation (BlueMax; Becton, Dickinson and Company, Japan) was charged with one tablet of test preparation, 50 g of glass beads BZ-4 (AS ONE Corporation) having an outer diameter of 3.962 mm to 4.699 mm, and 30 mL of the JP first fluid. The tube was horizontally laid and was rotated at 200 rpm at 37°C for 10 hours with the JP first fluid (30 mL) replaced every 2.5 hours. Thereafter, the small diameter of the gastric resident layer of the preparation was measured with a caliper. The test preparation thus obtained was then placed on a rheometer (Sun Rheo Meter CR-150; Sun Scientific Co., Ltd.), with the gastric resident layer on top and the drug releasing layer at the bottom. A maximum load was measured at a compression clearance of 1 mm and a measurement range of 10 kg, using a 3.5 cm flat circular compression adapter. Fig. 1 illustrates the apparatus used for the test.

### (Gastric residence time)

The test preparation was orally administered to groups of 2 to 3 beagles, 8 to 10 months of age, and the dogs were x-rayed over a time course to measure gastric residence time. Specifically, each group of beagles was put in a cage with free access to water. The dogs were fasted for at least 20 hours, and were given a mixture of 250 g of solid dog food and 50 g of chum 30 minutes before administration of the preparation. Upon confirmation that all the food has been eaten, the test preparation was administered together with 20 mL of water. This was followed by administration of 20 w/v% barium sulfate (10 mL/body), and the dogs were deprived of water until the end of the test. After administration of the test preparation, the subjects were x-rayed at one hour intervals, and 20 w/v% barium sulfate (10 mL/body) was administered as appropriate during the course of x-ray radiography. Note that, the test preparation was embedded with a circular tablet for x-ray imaging at each center of the drug releasing layer and the gastric resident layer, which tablet had been prepared from a mixture of barium sulfate (500 mg) and hydroxypropyl cellulose (HPC-H fine powder, 500 mg) using an IR-measurement tableting machine measuring 3 mm in diameter. The gastric residence time was given by the mean value of the time period from the administration of the test preparation to the last observation of the preparation in the stomach by x-ray radiography. For cases the preparation was observed in the stomach 16 hours after the administration but was not observed 24 hours after the administration, the gastric residence time was deemed as 16 hours.

### Reference Example 1

Furosemide (1250 mg), hydroxypropyl methyl cellulose 2208 (Metolose 90SH 4000SR, 3250 mg) and hydroxypropyl cellulose (HPC-M fine powder, 500 mg) were mixed together in a mortar to prepare a composition for the drug releasing layer. Separately, hydroxypropyl methyl cellulose acetate succinate (Shin-Etsu AQOAT AS-MF, 800 mg), hydroxypropyl cellulose (HPC-M fine powder, 200 mg) and yellow ferric oxide (5 mg) were mixed together in a mortar to prepare a composition for the gastric resident layer. The composition for the drug releasing layer and the composition for the gastric resident layer were loaded into a tableting machine (N-30E, Okada Seiko Co., Ltd.; hereinafter, the same machine will be used) in this order and the compositions were compressed into a tablet at a punch pressure of about 10 kN, using a circular die and punch measuring 8 mm in diameter. As a result, flat circular tablets of gastric retentive preparation having a diameter of 8 mm were prepared, each containing: furosemide (50 mg), hydroxypropyl methyl cellulose 2208 (130 mg) and hydroxypropyl cellulose (20 mg) for the drug releasing layer; and hydroxypropyl methyl cellulose acetate succinate (80 mg), hydroxypropyl cellulose (20 mg) and yellow ferric oxide (0.5 mg) for the gastric resident layer. Note that, when loading the composition for the drug releasing layer, the tablet for x-ray imaging was placed at the center of the composition after half of the composition had been loaded, and the rest of the composition was loaded thereon to embed the tablet for x-ray imaging into the drug releasing layer. In the same manner, when loading the composition for the gastric resident layer, the tablet for x-ray imaging was placed at the center of the composition after half of the composition had been loaded, and the rest of the composition was loaded thereon to embed the tablet for x-ray imaging in the gastric resident layer.

### (Dissolution test)

Small diameter (JP first fluid): 8.53 mm
Dissolution time (JP second fluid): 3.0 hours

### (Strength test)

Small diameter (JP first fluid) : 8. 32 mm, maximum load: > 10000 g

### (Confirmation test for gastric residence time)

Gastric residence time: 16 hours

### Reference Example 2

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and hydroxypropyl methyl cellulose acetate succinate (70 mg), lactose (30 mg) and yellow ferric oxide (0.5 mg) for the gastric resident layer.

### (Dissolution test)

Small diameter (JP first fluid): 8.42 mm
Dissolution time (JP second fluid): 1.0 hour

### (Strength test)

Small diameter (JP first fluid) : 8.23 mm, maximum load: > 10000 g

### (Confirmation test for gastric residence time)

Gastric residence time: 16 hours

### Reference Example 3

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and methacrylic acid-ethyl acrylate copolymer (Eudragit L100-55, 80 mg), hydroxypropyl cellulose (20 mg) and yellow ferric oxide (0.5 mg) for the gastric resident layer.

### (Dissolution test)

Small diameter (JP first fluid): 9.32 mm
Dissolution time (JP second fluid): 3.0 hours

### (Strength test)

Small diameter (JP first fluid) : 9.23 mm, maximum load: > 10000 g

### (Confirmation test for gastric residence time)

Gastric residence time: 16 hours

### Reference Example 4

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and methacrylic acid-ethyl acrylate copolymer (80 mg) and hydroxypropyl methyl cellulose 2208 (Metolose 90SH 100SR, 20 mg) for the gastric resident layer.

### (Dissolution test)

Small diameter (JP first fluid): 9.84 mm
Dissolution time (JP second fluid): 5.0 hours

### (Strength test)

Small diameter (JP first fluid) : 9.31 mm, maximum load: > 10000 g

### Reference Example 5

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and methacrylic acid-ethyl acrylate copolymer (80 mg) and hydroxypropyl methyl cellulose 2208 (Metolose 90SH 4000SR, 20 mg) for the gastric resident layer.

### (Dissolution test)

Small diameter (JP first fluid): 10.29 mm
Large diameter (JP second fluid): 1.71 mm

### (Strength test)

Small diameter (JP first fluid) : 9.41 mm, maximum load: > 10000 g

### Reference Example 6

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and methacrylic acid-ethyl acrylate copolymer (80 mg) and hydroxypropyl methyl cellulose 2208 (Metolose 90SH 100000SR, 20 mg) for the gastric resident layer.

### (Strength test)

Small diameter (JP first fluid): 9.65 mm, maximum load: > 10000 g

### Reference Example 7

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and methacrylic acid-ethyl acrylate copolymer (70 mg), hydroxypropyl methyl cellulose 2208 (Metolose 90SH 4000SR, 20 mg) and lactose (10 mg) for the gastric resident layer.

### (Dissolution test)

Small diameter (JP first fluid): 10.25 mm
Dissolution time (JP second fluid): 7.0 hours

### (Strength test)

Small diameter (JP first fluid): 9.62 mm, maximum load: 9760 g

### (Confirmation test for gastric residence time)

Gastric residence time: 19 hours

### Reference Example 8

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and methacrylic acid-ethyl acrylate copolymer (60 mg) and hydroxypropyl methyl cellulose 2208 (Metolose 90SH 4000SR, 40 mg) for the gastric resident layer.

### (Strength test)

Small diameter (JP first fluid): 9.34 mm, maximum load: 6490 g

### Reference Example 9

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and methacrylic acid-ethyl acrylate copolymer (70 mg) and hydroxypropyl methyl cellulose 2208 (Metolose 90SH 4000SR, 30 mg) for the gastric resident layer.

### (Dissolution test)

Small diameter (JP first fluid): 10.43 mm
Large diameter (JP second fluid): 1.82 mm

### (Strength test)

Small diameter (JP first fluid) : 9. 90 mm, maximum load: > 10000 g

### Reference Example 10

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and methacrylic acid-ethyl acrylate copolymer (40 mg) and hydroxypropyl cellulose (10 mg) for the gastric resident layer.

### (Dissolution test)

Small diameter (JP first fluid): 8.74 mm
Dissolution time (JP second fluid): 3.5 hours

### (Strength test)

Small diameter (JP first fluid): 8.79 mm, maximum load: 6130 g

### Reference Example 11

According to Reference Example 1, flat circular tablets of gastric retentive preparation having a diameter of 8 mm were prepared, each containing: ciprofloxacin (30 mg), polyethylene oxide (Polyox WSR-303, 85 mg) and hydroxypropyl cellulose (85 mg) for the drug releasing layer; and methacrylic acid-ethyl acrylate copolymer (35 mg) and hydroxypropyl methyl cellulose 2208 (Metolose 90SH 100SR, 10 mg) and low substituted hydroxypropyl cellulose (5 mg) for the gastric resident layer.

### (Dissolution test)

Small diameter (JP first fluid): 9.65 mm
Dissolution time (JP second fluid): 3.0 hours

### (Strength test)

Small diameter (JP first fluid): 9.21 mm, maximum load: 5140 g

### (Confirmation test for gastric residence time)

Gastric residence time: 15 hours

### Reference Example 12

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and hydrogenated oil (Lubriwax 101, hereinafter the same name will be used; 75 mg), carboxyvinyl polymer (5 mg), polyethylene oxide (Polyox WSR Coagulant; 5 mg) and anhydrous calcium hydrogen phosphate (15 mg) for the gastric resident layer.

### (Dissolution test)

Small diameter (JP first fluid): 8.00 mm
Dissolution time (JP second fluid): 8.0 hours

### (Strength test)

Small diameter (JP first fluid): 7.00 mm, maximum load: 6720 g

### (Confirmation test for gastric residence time)

Gastric residence time: 19 hours

### Reference Example 13

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and ethyl cellulose (Ethocel STD 10FP, 80 mg), carboxyvinyl polymer (10 mg) and polyethylene oxide (Polyox WSR 301, 10 mg) for the gastric resident layer.

### (Dissolution test)

Small diameter (JP first fluid): 8.82 mm
Large diameter (JP second fluid): 4.14 mm

### (Strength test)

Small diameter (JP first fluid): 8.47 mm, maximum load: 7750 g

### (Confirmation test for gastric residence time)

Gastric residence time: 16 hours

### Reference Example 14

According to Reference Example 1 except for using an irregular-shaped die and punch having a large diameter of 14 mm and a small diameter of 7 mm, irregular-shaped tablets of gastric retentive preparation having a large diameter of 14 mm and a small diameter of 7 mm were prepared, each containing: furosemide (50 mg), hydroxypropyl methyl cellulose 2208 (Metolose 90SH 4000SR, 150 mg), hydroxypropyl methyl cellulose 2910 (Metolose 60SH 50, 115 mg) and hydroxypropyl cellulose (35 mg) for the drug releasing layer; and hydrogenated oil (135 mg), carboxyvinyl polymer (7.5 mg) and crospovidone (Polyplasdone XL-10, 7.5 mg) for the gastric resident layer.

### (Dissolution test)

Small diameter (JP first fluid): 7.14 mm
Dissolution time (JP second fluid): 8 hours

### (Strength test)

Small diameter (JP first fluid) : 7.05 mm, maximum load: > 10000 g

### (Confirmation test for gastric residence time)

Gastric residence time: 19 hours

### Reference Example 15

According to Reference Example 1 except for using an irregular-shaped die and punch having a large diameter of 17.5 mm and a small diameter of 7.5 mm, irregular-shaped tablets of gastric retentive preparation having a large diameter of 17.5 mm and a small diameter of 7.5 mm were prepared, each containing: furosemide (50 mg), hydroxypropyl methyl cellulose 2208 (Metolose 90SH 4000SR, 150 mg), hydroxypropyl methyl cellulose 2910 (Metolose 60SH 50, 205 mg) and hydroxypropyl cellulose (45 mg) for the drug releasing layer; and methacrylic acid-ethyl acrylate copolymer (140 mg), hydroxypropyl cellulose (40 mg) and lactose (20 mg) for the gastric resident layer.

### (Dissolution test)

Small diameter (JP first fluid): 8.25 mm
Dissolution time (JP second fluid): 7 hours

### (Strength test)

Small diameter (JP first fluid) : 7.82 mm, maximum load: > 10000 g

### (Confirmation test for gastric residence time)

Gastric residence time: 16 hours

### Reference Example 16

According to Reference Example 1 except for using an irregular-shaped die and punch having a large diameter of 17.5 mm and a small diameter of 7.5 mm, irregular-shaped tablets of gastric retentive preparation having a large diameter of 17.5 mm and a small diameter of 7.5 mm were prepared, each containing: furosemide (50 mg), hydroxypropyl methyl cellulose 2208 (Metolose 90SH 4000SR, 150 mg), hydroxypropyl methyl cellulose 2910 (Metolose 60SH 50, 205 mg) and hydroxypropyl cellulose (45 mg) for the drug releasing layer; and methacrylic acid-ethyl acrylate copolymer (160 mg), hydroxypropyl methyl cellulose 2208 (Metolose 90SH 100SR, 40 mg) for the gastric resident layer.

### (Dissolution test)

Small diameter (JP first fluid): 8.98 mm
Dissolution time (JP second fluid): 4 hours

### (Strength test)

Small diameter (JP first fluid) : 8.55 mm, maximum load: > 10000 g

### (Confirmation test for gastric residence time)

Gastric residence time: 19 hours

### Reference Example 17

According to Reference Example 1 except for using an irregular-shaped die and punch having a large diameter of 17.5 mm and a small diameter of 7.5 mm, irregular-shaped tablets of gastric retentive preparation having a large diameter of 17.5 mm and a small diameter of 7.5 mm were prepared, each containing: furosemide (50 mg), hydroxypropyl methyl cellulose 2208 (Metolose 90SH 4000SR, 150 mg), hydroxypropyl methyl cellulose 2910 (Metolose 60SH 50, 205 mg) and hydroxypropyl cellulose (45 mg) for the drug releasing layer; and hydroxypropyl methyl cellulose acetate succinate (160 mg) and hydroxypropyl methyl cellulose 2208 (Metolose 90SH 100SR, 40 mg) for the gastric resident layer.

### (Dissolution test)

Small diameter (JP first fluid): 8.76 mm
Dissolution time (JP second fluid): 4 hours

### (Strength test)

Small diameter (JP first fluid) : 8.43 mm, maximum load: > 10000 g

### (Confirmation test for gastric residence time)

Gastric residence time: 17 hours

### Reference Example 18

According to Reference Example 1 except for using an irregular-shaped die and punch having a large diameter of 14 mm and a small diameter of 7 mm, irregular-shaped tablets of gastric retentive preparation having a large diameter of 14 mm and a small diameter of 7 mm were prepared, each containing: furosemide (50 mg), hydroxypropyl methyl cellulose 2208 (Metolose 90SH 4000SR, 150 mg), hydroxypropyl methyl cellulose 2910 (Metolose 60SH 50, 115 mg) and hydroxypropyl cellulose (35 mg) for the drug releasing layer; and methacrylic acid-ethyl acrylate copolymer (52.5 mg), hydroxypropyl methyl cellulose acetate succinate (52.5 mg), hydroxypropyl methyl cellulose 2208 (Metolose 90SH 100SR, 30 mg) and lactose (15 mg) for the gastric resident layer.

### (Dissolution test)

Small diameter (JP first fluid): 8.79 mm
Dissolution time (JP second fluid): 5 hours

### (Strength test)

Small diameter (JP first fluid): 8.35 mm, maximum load: 7940 g

### (Confirmation test for gastric residence time)

Gastric residence time: 18 hours

### Reference Example 19

According to Reference Example 1 except for using an irregular-shaped die and punch having a large diameter of 17.5 mm and a small diameter of 7.5 mm, irregular-shaped tablets of gastric retentive preparation having a large diameter of 17.5 mm and a small diameter of 7.5 mm were prepared, each containing: furosemide (50 mg), hydroxypropyl methyl cellulose 2208 (Metolose 90SH 4000SR, 150 mg), hydroxypropyl methyl cellulose 2910 (Metolose 60SH 50, 205 mg) and hydroxypropyl cellulose (45 mg) for the drug releasing layer; and hydroxypropyl methyl cellulose acetate succinate (160 mg) and hydroxypropyl cellulose (40 mg) for the gastric resident layer.

### (Dissolution test)

Small diameter (JP first fluid): 8.21 mm
Dissolution time (JP second fluid): 4 hours

### (Strength test)

Small diameter (JP first fluid) : 7.97 mm, maximum load: > 10000 g

### Reference Example 20

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and hydroxypropyl methyl cellulose acetate succinate (100 mg) for the gastric resident layer.

### (Dissolution test)

Small diameter (JP first fluid): 8.09 mm
Dissolution time (JP second fluid): 2.5 hours

### (Strength test)

Small diameter (JP first fluid) : 8.00 mm, maximum load: > 10000 g

### Reference Example 21

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and hydroxypropyl methyl cellulose acetate succinate (70 mg) and hydroxypropyl cellulose (30 mg) for the gastric resident layer.

### (Dissolution test)

Small diameter (JP first fluid): 9.00 mm
Dissolution time (JP second fluid): 8 hours

### (Strength test)

Small diameter (JP first fluid): 8.51 mm, maximum load: 6770 g

### Reference Example 22

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and hydroxypropyl methyl cellulose acetate succinate (60 mg), hydroxypropyl cellulose (5 mg) and lactose (35 mg) for the gastric resident layer.

### (Dissolution test)

Small diameter (JP first fluid): 8.38 mm
Dissolution time (JP second fluid): 2 hours

### (Strength test)

Small diameter (JP first fluid): 8.20 mm, maximum load: 8460 g

### Reference Example 23

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and methacrylic acid-ethyl acrylate copolymer (40 mg) and hydroxypropyl methyl cellulose 2208 (Metolose 90SH 100SR, 10 mg) for the gastric resident layer.

### (Dissolution test)

Small diameter (JP first fluid): 9.34 mm
Dissolution time (JP second fluid): 2 hours

### (Strength test)

Small diameter (JP first fluid): 9.35 mm, maximum load: 6790 g

### Reference Example 24

According to Reference Example 1, flat circular tablets of gastric retentive preparation having a diameter of 8 mm were prepared, each containing: ciprofloxacin (30 mg), polyethylene oxide (Polyox WSR-303, 85 mg) and hydroxypropyl cellulose (85 mg) for the drug releasing layer; and methacrylic acid-ethyl acrylate copolymer (35 mg), hydroxypropyl methyl cellulose 2208 (Metolose 90SH 100SR, 10 mg) and carmellose calcium (5 mg) for the gastric resident layer.

### (Dissolution test)

Small diameter (JP first fluid): 9.59 mm
Dissolution time (JP second fluid): 3 hours

### (Strength test)

Small diameter (JP first fluid): 8.96 mm, maximum load: 5120 g

### Reference Example 25

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and milled hydroxypropyl methyl cellulose phthalate 200731 (HPMCPHP-55, 80 mg) and hydroxypropyl cellulose (20 mg) for the gastric resident layer.

### (Dissolution test)

Small diameter (JP first fluid): 8.34 mm
Dissolution time (JP second fluid): 5 hours

### (Strength test)

Small diameter (JP first fluid) : 8.14 mm, maximum load: > 10000 g

### Reference Example 26

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and milled cellulose acetate phthalate (80 mg) and hydroxypropyl cellulose (20 mg) for the gastric resident layer.

### (Dissolution test)

Small diameter (JP first fluid): 8.31 mm
Dissolution time (JP second fluid): 2.5 hours

### (Strength test)

Small diameter (JP first fluid) : 7.82 mm, maximum load: > 10000 g

### Reference Example 27

According to Reference Example 1, flat circular tablets of gastric retentive preparation having a diameter of 8 mm were prepared, each containing: ciprofloxacin (30 mg), polyethylene oxide (Polyox WSR-303, 85 mg) and hydroxypropyl cellulose (85 mg) for the drug releasing layer; and hydrogenated oil (90 mg), polyethylene oxide (Polyox WSR N-750, 5 mg) and carmellose calcium (ECG-505, 5 mg) for the gastric resident layer.

### (Dissolution test)

Small diameter (JP first fluid): 8.03 mm
Long diameter (JP second fluid): 5.34 mm

### (Strength test)

Small diameter (JP first fluid) : 8.12 mm, maximum load: > 10000 g

### (Confirmation test for gastric residence time)

Gastric residence time: 14 hours

### Reference Example 28

According to Reference Example 1, flat circular tablets of gastric retentive preparation having a diameter of 8 mm were prepared, each containing: metformin (50 mg), hydroxypropyl methyl cellulose 2208 (Metolose 90SH 30000F, 130 mg) and hydroxypropyl cellulose (20 mg) for the drug releasing layer; and hydrogenated oil (80 mg), carboxyvinyl polymer (10 mg) and polyethylene oxide (Polyox WSR 301, 10 mg) for the gastric resident layer.

### (Dissolution test)

Small diameter (JP first fluid): 9.00 mm
Dissolution time (JP second fluid): 9 hours

### (Strength test)

Small diameter (JP first fluid): 8.80 mm, maximum load: 5160 g

### Reference Example 29

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and hydrogenated oil (70 mg), carboxyvinyl polymer (5 mg), polyethylene oxide (Polyox WSR coagulant, 5 mg) and anhydrous calcium hydrogen phosphate (20 mg) for the gastric resident layer.

### (Dissolution test)

Small diameter (JP first fluid): 8.27 mm
Large diameter (JP second fluid): 4.85 mm

### (Strength test)

Small diameter (JP first fluid) : 7.70 mm, maximum load: 5300 g

### Reference Example 30

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and hydrogenated oil (75 mg), carboxyvinyl polymer (10 mg), polyethylene oxide (Polyox WSR coagulant, 10 mg) and microcrystalline cellulose (Avicel PH 101, 5 mg) for the gastric resident layer.

### (Dissolution test)

Small diameter (JP first fluid): 9.21 mm
Large diameter (JP second fluid): 5.79 mm

### (Strength test)

Small diameter (JP first fluid): 8.70 mm, maximum load: 5180 g

### Reference Example 31

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and hydrogenated oil (80 mg), carboxyvinyl polymer (5 mg), polyethylene oxide (Polyox WSR Coagulant, 5 mg) and anhydrous calcium hydrogen phosphate (10 mg) for the gastric resident layer.

### (Dissolution test)

Small diameter (JP first fluid): 8.27 mm
Large diameter (JP second fluid): 4.84 mm

### (Strength test)

Small diameter (JP first fluid): 8.01 mm, maximum load: 8090 g

### Reference Example 32

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and hydrogenated oil (90 mg), carboxyvinyl polymer (5 mg) and polyethylene oxide (Polyox WSR -N750, 5 mg) for the gastric resident layer.

### (Dissolution test)

Small diameter (JP first fluid): 9.21 mm
Large diameter (JP second fluid): 4.13 mm

### (Strength test)

Small diameter (JP first fluid) : 8.10 mm, maximum load: > 10000 g

### Reference Example 33

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and hydrogenated oil (90 mg) and carboxyvinyl polymer (10 mg) for the gastric resident layer.

### (Dissolution test)

Small diameter (JP first fluid): 8.41 mm
Large diameter (JP second fluid): 4.77 mm

### (Strength test)

Small diameter (JP first fluid) : 8.26 mm, maximum load: > 10000 g

### Reference Example 34

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and hydrogenated oil (90 mg), polyethylene oxide (Polyox WSR-N750, 5 mg) and sodium carboxymethyl starch (Primogel, 5 mg) for the gastric resident layer.

### (Dissolution test)

Small diameter (JP first fluid): 8.08 mm
Large diameter (JP second fluid): 5.53 mm

### (Strength test)

Small diameter (JP first fluid) : 8.02 mm, maximum load: > 10000 g

### Reference Example 35

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and hydrogenated oil (90 mg), carboxyvinyl polymer (5 mg) and povidone (Plasdone K 29/32, 5 mg) for the gastric resident layer.

### (Dissolution test)

Small diameter (JP first fluid): 8.00 mm
Large diameter (JP second fluid): 4.97 mm

### (Strength test)

Small diameter (JP first fluid) : 8.05 mm, maximum load: > 10000 g

### Reference Example 36

According to Reference Example 1 except for using an irregular-shaped die and punch having a large diameter of 17.5 mm and a small diameter of 7.5 mm, irregular-shaped tablets of gastric retentive preparation having a large diameter of 17.5 mm and a small diameter of 7.5 mm were prepared, each containing: furosemide (50 mg), hydroxypropyl methyl cellulose 2208 (Metolose 90SH 4000SR, 150 mg), hydroxypropyl methyl cellulose 2910 (Metolose 60SH 50, 205 mg) and hydroxypropyl cellulose (45 mg) for the drug releasing layer; and xanthan gum (180 mg), hydroxypropyl methyl cellulose 2208 (Metolose 90SH 30000F, 157.5 mg), microcrystalline cellulose (76.5 mg) and guar gum (36 mg) for the gastric resident layer.

### (Dissolution test)

Small diameter (JP first fluid): 9.30 mm
Large diameter (JP second fluid): 21.74 mm

### (Strength test)

Small diameter (JP first fluid): 7.59 mm, maximum load: 4520 g

### (Confirmation test for gastric residence time)

Gastric residence time: 8 hours

Separately, the preparation was orally administered to beagles. The abdomen was opened 8 hours after the administration and the preparation remaining in the stomach was taken out to measure the small diameter of the gastric resident layer and maximum load of the preparation (*in vivo* strength test). Note that, the preparation was also treated in JP first fluid at 37°C for 8 hours to measure the small diameter and maximum load of the gastric resident layer according to the strength test described above (*in vitro* strength test).

### (in vivo strength test)

Small diameter: 7.18 mm; maximum load: 6370 g

### (in vitro strength test)

Small diameter: 8.49 mm; maximum load: 6460 g

From the result that the values of the *in vivo* strength test well coincide with the values of the *in vitro* strength test, it can be said that the *in vitro* strength test reflects the erosion of the preparation in the stomach.

### Reference Example 37

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and ethyl cellulose (Ethocel STD 10 FP, 50 mg), hydrogenated oil (50 mg) and yellow ferric oxide (0.5 mg) for the gastric resident layer.

### (Dissolution test)

Small diameter (JP first fluid): 8.00 mm
Large diameter (JP second fluid): 8.00 mm

### (Strength test)

Small diameter (JP first fluid): 8.00 mm, maximum load: > 10000 g

### (Confirmation test for gastric residence time)

Gastric residence time: 20 hours

### Reference Example 38

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and hydroxypropyl methyl cellulose 2208 (Metolose 90SH 100000SR, 100 mg) and yellow ferric oxide (0.5 mg) for the gastric resident layer.

### (Dissolution test)

Small diameter (JP first fluid): 9.45 mm
Large diameter (JP second fluid): 7.02 mm

### (Strength test)

Small diameter (JP first fluid): 7.15 mm, maximum load: 2570 g

### (Confirmation test for gastric residence time)

Gastric residence time: 8 hours

### Reference Example 39

According to Reference Example 1, flat circular tablets of gastric retentive preparation having a diameter of 8 mm were prepared, each containing: furosemide (50 mg), hydroxypropyl methyl cellulose 2208 (Metolose 90SH 4000SR, 130 mg) and hydroxypropyl cellulose (20 mg) for the drug releasing layer; and carboxyvinyl polymer (Carbopol 971P, 50 mg), polyethylene oxide (Polyox Coagulant, 50 mg) and yellow ferric oxide (0.5 mg) for the gastric resident layer.

### (Dissolution test)

Small diameter (JP first fluid): 10.68 mm
Large diameter (JP second fluid): 19.28 mm

### (Strength test)

Small diameter (JP first fluid) : 10.67 mm, maximum load: 2910 g

### (Confirmation test for gastric residence time)

Gastric residence time: 10 hours

### Reference Example 40

According to Reference Example 1 except for using a flat circular die and punch having a diameter of 6 mm, flat circular tablets of gastric retentive preparation having a diameter of 6 mm were prepared, each containing: furosemide (50 mg), hydroxypropyl methyl cellulose 2208 (Metolose 90SH 100000SR, 40 mg) and hydroxypropyl cellulose (10 mg) for the drug releasing layer; and ethyl cellulose (Ethocel STD 10FP, 12.5 mg), hydrogenated oil (12.5 mg), barium sulfate (25 mg) and yellow ferric oxide (0.5 mg) for the gastric resident layer.

### (Dissolution test)

Small diameter (JP first fluid): 6.00 mm
Large diameter (JP second fluid): 6.00 mm

### (Strength test)

Small diameter (JP first fluid): 6.00 mm, maximum load: 2020 g

### (Confirmation test for gastric residence time)

Gastric residence time: 6 hours

### Example 1

Levodopa (3100 mg), carbidopa (775 mg), hydroxypropyl methyl cellulose 2208 (Methocel K100M P CR, 1356.25 mg) and microcrystalline cellulose (Ceolus PH101, 193.75 mg) were mixed in a mortar to prepare a composition for a drug releasing layer. Separately, methacrylic acid-ethyl acrylate copolymer (Eudragit L100-55, 1860 mg) and hydroxypropyl methyl cellulose 2208 (Metolose 90SH 100SR, 465 mg) were mixed in a mortar to prepare a composition for a gastric resident layer. Firstly, the composition for a drug releasing layer was charged in a tableting machine and then the composition for a gastric resident layer was charged therein and subjected to a compressive molding with a tableting pressure of about 10 kN using an irregularly shaped die and punch where a large diameter was 14 mm and a small diameter was 7 mm to prepare a gastric retentive preparation in an irregularly shaped tablets of 14 mm large diameter and 7 mm small diameter containing: levodopa (200 mg), carbidopa (50 mg), hydroxypropyl methyl cellulose 2208 (87.5 mg) and microcrystalline cellulose (12.5 mg) in a drug releasing layer; and methacrylic acid-ethyl acrylate copolymer (120 mg) and hydroxypropyl methyl cellulose 2208 (30 mg) in a gastric resident layer per tablet.

### Example 2

According to Example 1, irregularly shaped tablets of gastric retentive preparation having a large diameter of 14 mm and a small diameter of 7 mm were prepared, each containing: levodopa (200 mg), carbidopa (50 mg), hydroxypropyl methyl cellulose 2208 (Methocel K100M P CR, 50 mg) and microcrystalline cellulose (50 mg) for the drug releasing layer; and methacrylic acid-ethyl acrylate copolymer (120 mg) and hydroxypropyl methyl cellulose 2208 (Metolose 90SH 100SR, 30 mg) for the gastric resident layer.

### Example 3

According to Example 1, irregularly shaped tablets of gastric retentive preparation having a large diameter of 14 mm and a small diameter of 7 mm were prepared, each containing: levodopa (200 mg), carbidopa (50 mg), hydroxypropyl methyl cellulose 2208 (Methocel K100M P CR, 75 mg) and microcrystalline cellulose (25 mg) for the drug releasing layer; and methacrylic acid-ethyl acrylate copolymer (120 mg) and hydroxypropyl methyl cellulose 2208 (Metolose 90SH 100SR, 30 mg) for the gastric resident layer.

### Example 4

According to Example 1, irregularly shaped tablets of gastric retentive preparation having a large diameter of 14 mm and a small diameter of 7 mm were prepared, each containing: levodopa (200 mg), carbidopa (50 mg) and hydroxypropyl methyl cellulose 2208 (Methocel K100M P CR, 100 mg) for the drug releasing layer; and methacrylic acid-ethyl acrylate copolymer (120 mg) and hydroxypropyl methyl cellulose 2208 (Metolose 90SH 100SR, 30 mg) for the gastric resident layer.

### (Dissolution test)

Small diameter (JP first fluid): 8.56 mm
Dissolution time (JP second fluid): 6 hours

### (Strength test)

Small diameter (JP first fluid) : 8.34 mm, maximum load: > 10000 g

### Example 5

According to Example 1 except for using an irregular-shaped die and punch having a large diameter of 17.5 mm and a small diameter of 7.5 mm, irregular-shaped tablets of gastric retentive preparation having a large diameter of 17.5 mm and a small diameter of 7.5 mm were prepared, each containing: levodopa (200 mg), carbidopa (50 mg), hydroxypropyl methyl cellulose 2208 (Methocel K100M P CR, 75 mg) and microcrystalline cellulose (125 mg) for the drug releasing layer; and methacrylic acid-ethyl acrylate copolymer (160 mg) and hydroxypropyl methyl cellulose 2208 (Metolose 90SH 100SR, 40 mg) for the gastric resident layer.

### Example 6

According to Example 1 except for using an irregular-shaped die and punch having a large diameter of 17.5 mm and a small diameter of 7.5 mm, irregular-shaped tablets of gastric retentive preparation having a large diameter of 17.5 mm and a small diameter of 7.5 mm were prepared, each containing: levodopa (200 mg), carbidopa (50 mg), hydroxypropyl methyl cellulose 2208 (Methocel K100M P CR, 100 mg) and microcrystalline cellulose (100 mg) for the drug releasing layer; and methacrylic acid-ethyl acrylate copolymer (160 mg) and hydroxypropyl methyl cellulose 2208 (Metolose 90SH 100SR, 40 mg) for the gastric resident layer.

### (Dissolution test)

Small diameter (JP first fluid): 9.46 mm
Dissolution time (JP second fluid): 5.8 hours

### (Strength test)

Small diameter (JP first fluid) : 8.89 mm, maximum load: > 10000 g

### Example 7

According to Example 1, irregularly shaped tablets of gastric retentive preparation having a large diameter of 14 mm and a small diameter of 7 mm were prepared, each containing: levodopa (200 mg), carboxyvinyl polymer (Carbopol 971P, 20 mg), anhydrous calcium hydrogen phosphate (20 mg) and hydroxypropyl methyl cellulose 2208 (Methocel K100M P CR, 150 mg) for the drug releasing layer; and methacrylic acid-ethyl acrylate copolymer (120 mg) and hydroxypropyl cellulose (HPC-M fine powder, 30 mg) for the gastric resident layer.

### (Dissolution test)

Small diameter (JP first fluid): 8.14 mm
Dissolution time (JP second fluid): 9 hours

### (Strength test)

Small diameter (JP first fluid) : 8.16 mm, maximum load: > 10000 g

### Example 8

Levodopa (2550 mg), carbidopa (255 mg), corn starch (255 mg) and povidone (Plasdone K29/32, 102 mg) were mixed in a mortar and water was added thereto to granulate. The granules were dried at 60°C and sieved to prepare granules of levodopa-carbidopa. To the levodopa-carbidopa granules (1922 mg) were added hydroxypropyl methyl cellulose 2208 (Methocel K100MP CR, 496 mg) and carboxyvinyl polymer (Carbopol 971P, 124 mg) followed by mixing in a glass bottle to prepare a composition for a drug releasing layer. Separately, methacrylic acid-ethyl acrylate copolymer (Eudragit L100-55, 1860 mg) and hydroxypropyl cellulose (HPC-M fine powder, 465 mg) were mixed in a mortar to prepare a composition for a gastric resident layer. Firstly, the composition for a drug releasing layer was charged in a tableting machine and then the composition for a gastric resident layer was charged therein and the mixture was subjected to a compressive molding with a tableting pressure of about 10 kN using an irregularly shaped die and punch where a large diameter was 14 mm and a small diameter was 7 mm to prepare a gastric retentive preparation in an irregularly shaped tablets of 14 mm large diameter and 7 mm small diameter containing: levodopa (250 mg), carbidopa (25 mg), corn starch (25 mg), povidone (10 mg), hydroxypropyl methyl cellulose 2208 (80 mg) and carboxyvinyl polymer (20 mg) for a drug releasing layer; and methacrylic acid-ethyl acrylate copolymer (120 mg) and hydroxypropyl cellulose (30 mg) for a gastric resident layer per tablet.

### Example 9

Levodopa (2040mg), carbidopa (510 mg), microcrystalline cellulose (Ceolus PH101, 102 mg), methacrylic acid-ethyl acrylate copolymer (Eudragit L100-55, 102 mg) and povidone (Plasdone K29/32, 102 mg) were mixed in a mortar and 99.5% ethanol was added thereto to granulate. The granules were dried at 60°C and sieved to prepare granules of levodopa-carbidopa. To the levodopa-carbidopa granules (1736 mg) was added hydroxypropyl methyl cellulose 2208 (Methocel K100MP CR, 620 mg) followed by mixing in a glass bottle to prepare a composition for a drug releasing layer. Separately, methacrylic acid-ethyl acrylate copolymer (Eudragit L100-55, 1860 mg) and hydroxypropyl cellulose (HPC-M fine powder, 465 mg) were mixed in a mortar to prepare a composition for a gastric resident layer. Firstly, the composition for a drug releasing layer was charged in a tableting machine and then the composition for a gastric resident layer was charged therein and the mixture was subjected to a compressive molding with a tableting pressure of about 10 kN using an irregularly shaped die and punch where a large diameter was 14 mm and a small diameter was 7 mm to prepare a gastric retentive preparation in an irregularly shaped tablets of 14 mm large diameter and 7 mm small diameter containing: levodopa (200 mg), carbidopa (50 mg), microcrystalline cellulose (10 mg), methacrylic acid-ethyl acrylate copolymer (10 mg), povidone (10 mg) and hydroxypropyl methyl cellulose 2208 (100 mg) for a drug releasing layer; and methacrylic acid-ethyl acrylate copolymer (120 mg) and hydroxypropyl cellulose (30 mg) for a gastric resident layer per tablet.

### Example 10

Levodopa (2040mg), carbidopa (510mg), microcrystalline cellulose (Ceolus PH101, 102 mg), hydroxypropyl methyl cellulose acetate succinate (HPMCAS-MF, 102mg) and povidone (Plasdone K29/32, 102 mg) were mixed in a mortar and 99.5% ethanol was added thereto to granulate. The granules were dried at 60°C and sieved to prepare granules of levodopa-carbidopa. To the levodopa-carbidopa granules (1736 mg) was added hydroxypropyl methyl cellulose 2208 (Methocel K100MP CR, 620 mg) followed by mixing in a glass bottle to prepare a composition for a drug releasing layer. Separately, hydroxypropyl methyl cellulose acetate succinate (HPMCAS-MF, 1627.5 mg) and lactose (697.5 mg) were mixed in a mortar to prepare a composition for a gastric resident layer. Firstly, the composition for a drug releasing layer was charged in a tableting machine and then the composition for a gastric resident layer was charged therein and the mixture was subjected to a compressive molding with a tableting pressure of about 10 kN using an irregularly shaped die and punch where a large diameter was 14 mm and a small diameter was 7 mm to prepare a gastric retentive preparation in an irregularly shaped tablets of 14 mm large diameter and 7 mm small diameter containing: levodopa (200 mg), carbidopa (50 mg), microcrystalline cellulose (10 mg), hydroxypropyl methyl cellulose acetate succinate (10 mg), povidone (10 mg) and hydroxypropyl methyl cellulose 2208 (100 mg) for a drug releasing layer; and hydroxypropyl methyl cellulose acetate succinate (105 mg) and lactose (45 mg) for a gastric resident layer per tablet.

### Example 11

Levodopa (2040 mg), carbidopa (510 mg), microcrystalline cellulose (Ceolus PH101, 102 mg), xanthan gum (102 mg) and povidone(Plasdone K29/32, 102 mg) were mixed in a mortar and water was added thereto to granulate. The granules were dried at 60°C and sieved to prepare granules of levodopa-carbidopa. To the levodopa-carbidopa granules (1736 mg) was added hydroxypropyl methyl cellulose 2208 (Methocel K100MP CR, 620 mg) followed by mixing in a glass bottle to prepare a composition for a drug releasing layer. Separately, hydroxypropyl methyl cellulose acetate succinate (HPMCAS-MF, 1627.5 mg) and lactose (697.5 mg) were mixed in a mortar to prepare a composition for a gastric resident layer. Firstly, the composition for a drug releasing layer was charged in a tableting machine and then the composition for a gastric resident layer was charged therein and the mixture was subjected to a compressive molding with a tableting pressure of about 10 kN using an irregularly shaped die and punch where a large diameter was 14 mm and a small diameter was 7 mm to prepare a gastric retentive preparation in an irregularly shaped tablets of 14 mm large diameter and 7 mm small diameter containing: levodopa (200 mg), carbidopa (50 mg), microcrystalline cellulose (10 mg), xanthan gum (10 mg), povidone (10 mg) and hydroxypropyl methyl cellulose 2208 (100 mg) for a drug releasing layer; and hydroxypropyl methyl cellulose acetate succinate (105 mg) and lactose (45 mg) for a gastric resident layer per tablet.

### Example 12

Levodopa (2040mg), carbidopa (510 mg), microcrystalline cellulose (Ceolus PH101, 102 mg), gellan gum (102 mg) and povidone (Plasdone K29/32, 102 mg) were mixed in a mortar and water was added thereto to granulate. The granules were dried at 60°C and sieved to prepare granules of levodopa-carbidopa. To the levodopa-carbidopa granules (1736 mg) was added hydroxypropyl methyl cellulose 2208 (Methocel K100MP CR, 620 mg) followed by mixing in a glass bottle to prepare a composition for a drug releasing layer. Separately, hydroxypropyl methyl cellulose acetate succinate (HPMCAS-MF, 1627.5 mg) and lactose (697.5 mg) were mixed in a mortar to prepare a composition for a gastric resident layer. Firstly, the composition for a drug releasing layer was charged in a tableting machine and then the composition for a gastric resident layer was charged therein and the mixture was subjected to a compressive molding with a tableting pressure of about 10 kN using an irregularly shaped die and punch where a large diameter was 14 mm and a small diameter was 7 mm to prepare a gastric retentive preparation in an irregularly shaped tablets of 14 mm large diameter and 7 mm small diameter containing: levodopa (200 mg), carbidopa (50 mg), microcrystalline cellulose (10 mg), gellan gum (10 mg), povidone (10 mg) and hydroxypropyl methyl cellulose 2208 (100 mg) for a drug releasing layer; and hydroxypropyl methyl cellulose acetate succinate (105 mg) and lactose (45 mg) for a gastric resident layer per tablet.

### Example 13

Levodopa (8200 mg), carbidopa (2050 mg), microcrystalline cellulose (410 mg) and carboxyvinyl polymer (Carbopol 971P, 410 mg) were mixed in a mortar and water was added thereto to granulate. The granules were dried at 60°C and sieved to prepare granules of levodopa-carbidopa. To the levodopa-carbidopa granules (1674 mg) was added hydroxypropyl methyl cellulose 2208 (Methocel K100MP CR, 620 mg) followed by mixing in a glass bottle to prepare a composition for a drug releasing layer. Separately, methacrylic acid-ethyl acrylate copolymer (Eudragit L100-55, 1076.25 mg), hydroxypropyl methyl cellulose acetate succinate (HPMCAS-MF, 1076.25 mg), hydroxypropyl methyl cellulose 2208 (Metolose 90SH 100SR, 615 mg) and lactose (307.5 mg) were mixed in a mortar to prepare a composition for a gastric resident layer. Firstly, the composition for a drug releasing layer was charged in a tableting machine and then the composition for a gastric resident layer was charged therein and the mixture was subjected to a compressive molding with a tableting pressure of about 10 kN using an irregularly shaped die and punch where a large diameter was 14 mm and a small diameter was 7 mm to prepare a gastric retentive preparation in an irregularly shaped tablets of 14 mm large diameter and 7 mm small diameter containing: levodopa (200 mg), carbidopa (50 mg), microcrystalline cellulose (10 mg), carboxyvinyl polymer (10 mg) and hydroxypropyl methyl cellulose 2208 (100 mg) for a drug releasing layer; and methacrylic acid-ethyl acrylate copolymer (52.5 mg), hydroxypropyl methyl cellulose acetate succinate (52.5 mg), hydroxypropyl methyl cellulose 2208 (30 mg) and lactose (15 mg) for a gastric resident layer per tablet.

### (Dissolution test)

Small diameter (JP first fluid): 8.67 mm
Dissolution time (JP second fluid): 4 hours

### (Strength test)

Small diameter (JP first fluid): 8.23 mm, maximum load: 7055 g

### Example 14

According to Example 13, granules of levodopa-carbidopa were prepared. To the levodopa-carbidopa granules (1674 mg) was added polyethylene oxide (Polyox WSR 303, 620 mg) followed by mixing in a glass bottle to prepare a composition for a drug releasing layer. Separately, methacrylic acid-ethyl acrylate copolymer (Eudragit L100-55, 2152.5 mg), hydroxypropyl methyl cellulose 2208 (Metolose 90SH 4000SR, 615 mg) and lactose (307.5 mg) were mixed in a mortar to prepare a composition for a gastric resident layer. Firstly, the composition for a drug releasing layer was charged in a tableting machine and then the composition for a gastric resident layer was charged therein and the mixture was subjected to a compressive molding with a tableting pressure of about 10 kN using an irregularly shaped die and punch where a large diameter was 14 mm and a small diameter was 7 mm to prepare a gastric retentive preparation in an irregularly shaped tablets of 14 mm large diameter and 7 mm small diameter containing: levodopa (200 mg), carbidopa (50 mg), microcrystalline cellulose (10 mg), carboxyvinyl polymer (10 mg) and polyethylene oxide (100 mg) for a drug releasing layer; and methacrylic acid-ethyl acrylate copolymer (105 mg), hydroxypropyl methyl cellulose 2208 (30 mg) and lactose (15 mg) for a gastric resident layer per tablet.

### (Dissolution test)

Small diameter (JP first fluid): 10.29 mm
Dissolution time (JP second fluid): 4.3 hours

### (Strength test)

Small diameter (JP first fluid): 9.43 mm, maximum load: 9945 g

### Example 15

According to Example 13, granules of levodopa-carbidopa were prepared. To the levodopa-carbidopa granules (1674 mg) was added hydroxypropyl cellulose (HPC-M fine powder, 620 mg) followed by mixing in a glass bottle to prepare a composition for a drug releasing layer. Separately, hydrogenated oil (2767.5 mg), carboxyvinyl polymer (Carbopol 971P, 153.75 mg) and crospovidone (Polyplasdone XL-10, 153.75 mg) were mixed in a mortar to prepare a composition for a gastric resident layer. Firstly, the composition for a drug releasing layer was charged in a tableting machine and then the composition for a gastric resident layer was charged therein and the mixture was subjected to a compressive molding with a tableting pressure of about 10 kN using an irregularly shaped die and punch where a large diameter was 14 mm and a small diameter was 7 mm to prepare a gastric retentive preparation in an irregularly shaped tablets of 14 mm large diameter and 7 mm small diameter containing: levodopa (200 mg), carbidopa (50 mg), microcrystalline cellulose (10 mg), carboxyvinyl polymer (10 mg) and hydroxypropyl cellulose (100 mg) for a drug releasing layer; and carboxyvinyl polymer (7.5 mg) and crospovidone (7.5 mg) for a gastric resident layer per tablet.

### (Dissolution test)

Small diameter (JP first fluid): 7.25 mm
Dissolution time (JP second fluid): 7 hours

### (Strength test)

Small diameter (JP first fluid): 7.05 mm, maximum load: 9505 g

### Example 16

According to Example 13, granules of levodopa-carbidopa were prepared. To the levodopa-carbidopa granules (1674 mg) were added hydroxypropyl methyl cellulose 2208 (Methocel K100MP CR, 496 mg) and carboxyvinyl polymer (Carbopol 971P, 124 mg) followed by mixing in a glass bottle to prepare a composition for a drug releasing layer. Separately, hydroxypropyl methyl cellulose acetate succinate (HPMCAS-MF, 2460 mg) and hydroxypropyl cellulose (HPC-M fine powder, 615 mg) were mixed in a mortar to prepare a composition for a gastric resident layer. Firstly, the composition for a drug releasing layer was charged in a tableting machine and then the composition for a gastric resident layer was charged therein and the mixture was subjected to a compressive molding with a tableting pressure of about 10 kN using an irregularly shaped die and punch where a large diameter was 14 mm and a small diameter was 7 mm to prepare a gastric retentive preparation in an irregularly shaped tablets of 14 mm large diameter and 7 mm small diameter containing: levodopa (200 mg), carbidopa (50 mg), microcrystalline cellulose (10 mg), carboxyvinyl polymer (30 mg) and hydroxypropyl methyl cellulose 2208 (80 mg) for a drug releasing layer; and hydroxypropyl methyl cellulose acetate succinate (120 mg) and hydroxypropyl cellulose (30 mg) for a gastric resident layer per tablet.

### (Dissolution test)

Small diameter (JP first fluid): 7.39 mm
Dissolution time (JP second fluid): 9 hours

### (Strength test)

Small diameter (JP first fluid): 7.34 mm, maximum load: 9350 g

### Example 17

According to Example 13, granules of levodopa-carbidopa were prepared. To the levodopa-carbidopa granules (1674 mg) were added polyethylene oxide (Polyox WSR 303, 310 mg) and carboxyvinyl polymer (Carbopol 971P, 310 mg) followed by mixing in a glass bottle to prepare a composition for a drug releasing layer. Separately, hydrogenated oil (2306.25 mg), carboxyvinyl polymer (153.75 mg), polyethylene oxide (Polyox Coagulant, 153.75 mg) and anhydrous calcium hydrogen phosphate (461.25 mg) were mixed in a mortar to prepare a composition for a gastric resident layer. Firstly, the composition for a drug releasing layer was charged in a tableting machine and then the composition for a gastric resident layer was charged therein and the mixture was subjected to a compressive molding with a tableting pressure of about 10 kN using an irregularly shaped die and punch where a large diameter was 14 mm and a small diameter was 7 mm to prepare a gastric retentive preparation in an irregularly shaped tablets of 14 mm large diameter and 7 mm small diameter containing: levodopa (200 mg), carbidopa (50 mg), microcrystalline cellulose (10 mg), carboxyvinyl polymer (60 mg) and polyethylene oxide (50 mg) for a drug releasing layer; and hydrogenated oil (112.5 mg), carboxyvinyl polymer (7.5 mg), polyethylene oxide (7. 5 mg) and anhydrous calcium hydrogen phosphate (22.5 mg) for a gastric resident layer per tablet.

### (Dissolution test)

Small diameter (JP first fluid): 7.13 mm
Dissolution time (JP second fluid): 9 hours

### (Strength test)

Small diameter (JP first fluid): 7.37 mm, maximum load: 8300 g

### Example 18

According to Example 13, granules of levodopa-carbidopa were prepared. To the levodopa-carbidopa granules (1674 mg) were added hydrogenated oil (496 mg) and lactose (124 mg) followed by mixing in a glass bottle to prepare a composition for a drug releasing layer. Separately, ethyl cellulose (Ethocel STD 10FP, 2460 mg), carboxyvinyl polymer (Carbopol 971P, 307.5 mg) and polyethylene oxide (Polyox WSR 301, 307.5 mg) were mixed in a mortar to prepare a composition for a gastric resident layer. Firstly, the composition for a drug releasing layer was charged in a tableting machine and then the composition for a gastric resident layer was charged therein and the mixture was subjected to a compressive molding with a tableting pressure of about 10 kN using an irregularly shaped die and punch where a large diameter was 14 mm and a small diameter was 7 mm to prepare a gastric retentive preparation in an irregularly shaped tablets of 14 mm large diameter and 7 mm small diameter containing: levodopa (200 mg), carbidopa (50 mg), microcrystalline cellulose (10 mg), carboxyvinyl polymer (10 mg), hydrogenated oil (80 mg) and lactose (20 mg) for a drug releasing layer; and ethyl cellulose (120 mg), carboxyvinyl polymer (15 mg) and polyethylene oxide (15 mg) for a gastric resident layer per tablet.

### (Dissolution test)

Small diameter (JP first fluid): 7.27 mm
Dissolution time (JP second fluid): 9 hours

### (Strength test)

Small diameter (JP first fluid) : 7. 30 mm, maximum load: > 10000 g

### Example 19

According to Example 1, there was prepared a gastric retentive preparation in three-layered (where a gastric resident layer is a middle layer) and irregular shaped tablets each having 14 mm large diameter and 7 mm small diameter and containing: levodopa (100 mg), carbidopa (25 mg), hydroxypropyl methyl cellulose 2208 (Methocel K100M P CR, 75 mg), microcrystalline cellulose (50 mg) and anhydrous citric acid (12.5 mg) for a sustained release drug releasing layer; methacrylic acid-ethyl acrylate copolymer (120 mg) and hydroxypropyl methyl cellulose 2208 (30 mg) for a gastric resident layer; and levodopa (100 mg), carbidopa (25 mg), low substituted hydroxypropyl cellulose (L-HPC LH-11, 7.5 mg), microcrystalline cellulose (15 mg) and anhydrous citric acid (12.5 mg) for an immediately release drug-releasing layer per tablet.

### Example 20

According to Example 19, there was prepared a gastric retentive preparation in three-layered and irregular shaped tablets each having 14 mm large diameter and 7 mm small diameter and containing: levodopa (140 mg), carbidopa (35 mg), hydroxypropyl methyl cellulose 2208 (Methocel K100M P CR, 87. 5 mg), microcrystalline cellulose (37.5 mg) and anhydrous citric acid (17.5 mg) for a sustained release drug releasing layer; methacrylic acid-ethyl acrylate copolymer (120 mg) and hydroxypropyl methyl cellulose 2208 (30 mg) for a gastric resident layer; and levodopa (60 mg), carbidopa (15 mg), low substituted hydroxypropyl cellulose (L-HPC LH-11, 4.5 mg), microcrystalline cellulose (9 mg) and anhydrous citric acid (7.5 mg) for an immediately release drug-releasing layer per tablet.

### Example 21

According to Example 19, there was prepared a gastric retentive preparation in three-layered and irregular shaped tablets each having 14 mm large diameter and 7 mm small diameter and containing: levodopa (180 mg), carbidopa (45 mg), hydroxypropyl methyl cellulose 2208 (Methocel K100M P CR, 100 mg), microcrystalline cellulose (25 mg) and anhydrous citric acid (22.5 mg) for a sustained release drug releasing layer; methacrylic acid-ethyl acrylate copolymer (120 mg) and hydroxypropyl methyl cellulose 2208 (30 mg) for a gastric resident layer; and levodopa (20 mg), carbidopa (5 mg), low substituted hydroxypropyl cellulose (L-HPC LH-11, 1.5 mg), microcrystalline cellulose (3 mg) and anhydrous citric acid (2.5 mg) for an immediately release drug-releasing layer per tablet.

### Example 22

According to Example 1, flat and circular tablets of gastric retentive preparation having a diameter of 8 mm were prepared, each containing: levodopa (100 mg), carbidopa (25 mg) and hydroxypropyl methyl cellulose 2208 (Methocel K100M P CR, 50 mg) for the sustained release drug releasing layer; and hydroxypropyl methyl cellulose acetate succinate (100 mg) for the gastric resident layer.

### (Dissolution test)

Small diameter (JP first fluid): 8.05 mm
Dissolution time (JP second fluid): 3 hours

### (Strength test)

Small diameter (JP first fluid) : 8.09 mm, maximum load: > 10000 g

### Example 23

According to Example 1, flat and circular tablets of gastric retentive preparation having a diameter of 8 mm were prepared, each containing: levodopa (100 mg), carbidopa (25 mg) and hydroxypropyl methyl cellulose 2208 (Methocel K100M P CR, 50 mg) for the sustained release drug releasing layer; and milled hydroxypropyl methyl cellulose phthalate 200731 (HPMCPHP-55, 80 mg) and hydroxypropyl cellulose (20 mg) for the gastric resident layer.

### (Dissolution test)

Small diameter (JP first fluid): 8.67 mm
Dissolution time (JP second fluid): 3 hours

### (Strength test)

Small diameter (JP first fluid): 7.87 mm, maximum load: 6080 g

### Example 24

According to Example 1, flat and circular tablets of gastric retentive preparation having a diameter of 8 mm were prepared, each containing: levodopa (100 mg), carbidopa (25 mg) and hydroxypropyl methyl cellulose 2208 (Methocel K100M P CR, 50 mg) for the sustained release drug releasing layer; and milled cellulose acetate phthalate (80 mg) and hydroxypropyl cellulose (20 mg) for the gastric resident layer.

### (Dissolution test)

Small diameter (JP first fluid): 8.39 mm
Dissolution time (JP second fluid): 3 hours

### (Strength test)

Small diameter (JP first fluid) : 8. 06 mm, maximum load: > 10000 g

### Example 25

According to Example 1, flat and circular tablets of gastric retentive preparation having a diameter of 8 mm were prepared, each containing: levodopa (100 mg), carbidopa (25 mg) and hydroxypropyl methyl cellulose 2208 (Methocel K100M P CR, 50 mg) for the sustained release drug releasing layer; and hydrogenated oil (75 mg), polyethylene oxide (Polyox WSR N-750, 5 mg), carmellose calcium (ECG-505, 5 mg) and microcrystalline cellulose (Ceolus PH101, 15 mg) for the gastric resident layer.

### (Dissolution test)

Small diameter (JP first fluid): 8.16 mm
Dissolution time (JP second fluid): 8 hours

### (Strength test)

Small diameter (JP first fluid): 7.62 mm, maximum load: 8885 g

### Example 26

According to Example 1, flat and circular tablets of gastric retentive preparation having a diameter of 8 mm were prepared, each containing: levodopa (100 mg), carbidopa (25 mg) and hydroxypropyl methyl cellulose 2208 (Methocel K100M P CR, 50 mg) for the sustained release drug releasing layer; and stearic acid (75 mg), carboxyvinyl polymer (5 mg), polyethylene oxide (Polyox WSR N-750, 5 mg) and anhydrous calcium hydrogen phosphate (15 mg) for the gastric resident layer.

### (Dissolution test)

Small diameter (JP first fluid): 8.72 mm
Dissolution time (JP second fluid): 9 hours

### (Strength test)

Small diameter (JP first fluid): 7.48 mm, maximum load: 6030 g

### Example 27

According to Example 1, irregular shaped tablets of gastric retentive preparation having a large diameter of 14 mm and a small diameter of 7 mm were prepared, each containing: levodopa (200 mg), carbidopa (50 mg), entacapone (200 mg) and hydroxypropyl methyl cellulose 2208 (Methocel K100M P CR, 100 mg) for the sustained release drug releasing layer; and methacrylic acid-ethyl acrylate copolymer (120 mg) and hydroxypropyl methyl cellulose 2208 (Metolose 90SH 100SR, 30 mg) for the gastric resident layer.

### Example 28

According to Example 1, irregular shaped tablets of gastric retentive preparation having a large diameter of 14 mm and a small diameter of 7 mm were prepared, each containing: levodopa (200 mg), carbidopa (50 mg), tolcapone (200 mg) and hydroxypropyl methyl cellulose 2208 (Methocel K100M P CR, 100 mg) for the sustained release drug releasing layer; and methacrylic acid-ethyl acrylate copolymer (120 mg) and hydroxypropyl methyl cellulose 2208 (Metolose 90SH 100SR, 30 mg) for the gastric resident layer.

### Example 29

According to Example 1, irregular shaped tablets of gastric retentive preparation having a large diameter of 14 mm and a small diameter of 7 mm were prepared, each containing: levodopa (200 mg), benserazide (50 mg) and hydroxypropyl methyl cellulose 2208 (Methocel K100M P CR, 100 mg) for the sustained release drug releasing layer; and methacrylic acid-ethyl acrylate copolymer (120 mg) and hydroxypropyl methyl cellulose 2208 (Metolose 90SH 100SR, 30 mg) for the gastric resident layer.

### Example 30

According to Example 1, flat and circular tablets of gastric retentive preparation having a diameter of 8 mm were prepared, each containing: levodopa (100 mg), carbidopa (25 mg) and hydroxypropyl methyl cellulose 2208 (Methocel K100M P CR, 50 mg) for the sustained release drug releasing layer; and hydroxypropyl methyl cellulose acetate succinate (60 mg), hydroxypropyl cellulose (5 mg) and lactose (35 mg) for the gastric resident layer.

### (Dissolution test)

Small diameter (JP first fluid): 8.40 mm
Dissolution time (JP second fluid): 1.5 hours

### (Strength test)

Small diameter (JP first fluid) : 8.28 mm, maximum load: 8870 g

### Example 31

According to Example 1, flat and circular tablets of gastric retentive preparation having a diameter of 8 mm were prepared, each containing: levodopa (100 mg), carbidopa (25 mg) and hydroxypropyl methyl cellulose 2208 (Methocel K100M P CR, 50 mg) for the sustained release drug releasing layer; and polyethylene oxide (Polyox WSR-N 750, 5 mg), sodium carboxymethyl starch (Primojel, 5 mg) and lactose (5 mg) for the gastric resident layer.

### (Dissolution test)

Small diameter (JP first fluid): 8.14 mm
Dissolution time (JP second fluid): 9 hours

### (Strength test)

Small diameter (JP first fluid): 7.88 mm, maximum load: > 10000 g

### Example 32

According to Example 1, flat and circular tablets of gastric retentive preparation having a diameter of 8 mm were prepared, each containing: levodopa (100 mg), carbidopa (25 mg) and hydroxypropyl methyl cellulose 2208 (Methocel K100M P CR, 50 mg) for the sustained release drug releasing layer; and hydrogenated oil (85 mg), xanthan gum (5 mg), polyethylene oxide (Polyox WSR-N 750, 5 mg) and anhydrous calcium hydrogen phosphate (5 mg) for the gastric resident layer.

### (Dissolution test)

Small diameter (JP first fluid): 8.06 mm
Dissolution time (JP second fluid): 5 hours

### (Strength test)

Small diameter (JP first fluid): 7.00 mm, maximum load: 6530 g

### Example 33

According to Example 1, flat and circular tablets of gastric retentive preparation having a diameter of 8 mm were prepared, each containing: levodopa (100 mg), carbidopa (25 mg) and hydroxypropyl methyl cellulose 2208 (Methocel K100M P CR, 50 mg) for the sustained release drug releasing layer; and methacrylic acid-ethyl acrylate copolymer (Eudragit L100-55, 70 mg), povidone (Plasdone K29/32, 10 mg), methyl cellulose (SM-4, 10 mg) and mannitol (10 mg) for the gastric resident layer.

### (Dissolution test)

Small diameter (JP first fluid): 9.16 mm
Dissolution time (JP second fluid): 3 hours

### (Strength test)

Small diameter (JP first fluid) : 8.77 mm, maximum load: > 10000 g

### Example 34

According to Example 1, flat and circular tablets of gastric retentive preparation having a diameter of 8 mm were prepared, each containing: levodopa (100 mg), carbidopa (25 mg) and hydroxypropyl methyl cellulose 2208 (Methocel K100M P CR, 50 mg) for the sustained release drug releasing layer; and methacrylic acid-ethyl acrylate copolymer (Eudragit L100-55, 70 mg), povidone (Plasdone K29/32, 10 mg), Macrogol 6000 (10 mg) and mannitol (10 mg) for the gastric resident layer.

### (Dissolution test)

Small diameter (JP first fluid): 9.96 mm
Dissolution time (JP second fluid): 2 hours

### (Strength test)

Small diameter (JP first fluid): 9.09 mm, maximum load: 6210 g

### Example 35

According to Example 1, flat and circular tablets of gastric retentive preparation having a diameter of 8 mm were prepared, each containing: levodopa (100 mg), carbidopa (25 mg) and hydroxypropyl methyl cellulose 2208 (Methocel K100M P CR, 50 mg) for the sustained release drug releasing layer; and hydrogenated oil (50 mg), stearyl alcohol (30 mg), carboxyvinyl polymer (5 mg), povidone (Plasdone K29/32, 5 mg), methyl cellulose (SM-4, 5 mg) and mannitol (5 mg) for the gastric resident layer.

### (Dissolution test)

Small diameter (JP first fluid): 8.24 mm
Dissolution time (JP second fluid): 9 hours

### (Strength test)

Small diameter (JP first fluid) : 8. 18 mm, maximum load: > 10000 g

### Example 36

According to Example 1, flat and circular tablets of gastric retentive preparation having a diameter of 8 mm were prepared, each containing: levodopa (100 mg), carbidopa (25 mg) and hydroxypropyl methyl cellulose 2208 (Methocel K100M P CR, 50 mg) for the sustained release drug releasing layer; and hydrogenated oil (50 mg), tetraglycerol pentaester (PS-3S, 30 mg), carboxyvinyl polymer (5 mg), povidone (Plasdone K29/32, 5 mg), Macrogol 6000 (5 mg) and mannitol (5 mg) for the gastric resident layer.

### (Dissolution test)

Small diameter (JP first fluid): 8.15 mm
Dissolution time (JP second fluid): 6 hours

### (Strength test)

Small diameter (JP first fluid) : 8.09 mm, maximum load: > 10000 g

### Example 37

According to Example 1, flat and circular tablets of gastric retentive preparation having a diameter of 8 mm were prepared, each containing: levodopa (100 mg), carbidopa (25 mg) and hydroxypropyl methyl cellulose 2208 (Methocel K100M P CR, 50 mg) for the sustained release drug releasing layer; and hydrogenated oil (60 mg), carboxyvinyl polymer (5 mg), povidone (Plasdone K29/32, 5 mg) and mannitol (30 mg) for the gastric resident layer.

### (Dissolution test)

Small diameter (JP first fluid): 8.10 mm
Dissolution time (JP second fluid): 6 hours

### (Strength test)

Small diameter (JP first fluid): 8.07 mm, maximum load: 9100 g

### Comparative Example 1

Levodopa (1600 mg) and carbidopa (400 mg) were dissolved in the JP first fluid (400 mL) to prepare a levodopa-carbidopa solution.

### Comparative Example 2

According to Example 1 except for using an irregular-shaped die and punch having a large diameter of 17.5 mm and a small diameter of 7.5 mm, irregular-shaped tablets of gastric retentive preparation having a large diameter of 17.5 mm and a small diameter of 7.5 mm were prepared, each containing: levodopa (200 mg), carbidopa (50 mg), hydroxypropyl methyl cellulose 2208 (Methocel K100M P CR, 100 mg) and microcrystalline cellulose (Ceolus PH 101, 100 mg) for the sustained release drug releasing layer; and xanthan gum (180 mg), hydroxypropyl methyl cellulose 2208 (Metolose 90SH 30000F, 157.5 mg), microcrystalline cellulose (76.5 mg) and guar gum (36 mg) for the gastric resident layer.

### (Dissolution test)

Small diameter (JP first fluid): 8.38 mm
Large diameter (JP second fluid): 22.14 mm

### (Strength test)

Small diameter (JP first fluid) : 7.76 mm, maximum load: 3000 g

### (Pharmacokinetic test in dogs)

Pharmacokinetic test was conducted in dogs using the preparations manufactured in Examples and Comparative Example, a levodopa-carbidopa solution and commercially available Sinemet CR tablets.

**[Table 1]**

| Test Nos. | Preparations Administered | Size of Preparation | Total Dose (Levodopa / Carbidopa) |
|---|---|---|---|
| 1 | Example 1 | 14 mm × 7 mm | 200 mg / 50 mg |
| 2 | Example 2 | 14 mm × 7 mm | 200 mg / 50 mg |
| 3 | Example 3 | 14 mm × 7 mm | 200 mg / 50 mg |
| 4 | Example 4 | 14 mm × 7 mm | 200 mg / 50 mg |
| 5 | Examples 4 + 2 (at the same time) | 14 mm × 7 mm | 400 mg / 100 mg |
| 6 | Example 5 | 17.5 mm × 7.5 mm | 200 mg / 50 mg |
| 7 | Example 6 | 17.5 mm × 7.5 mm | 200 mg / 50 mg |
| 8 | Comp Ex 1 | (solution) | 200 mg / 50 mg |
| 9 | Comp Ex 2 | 17.5 mm × 7.5 mm | 200 mg / 50 mg |
| 10 | Sinemet CR | 12.8 mm × 7.2 mm | 200 mg / 50 mg |
| 11 | Sinemet CR (additionally administered after 6 hrs) | 12.8 mm × 7.2 mm | 400 mg / 100 mg |

### (Measurement of drug concentrations in blood)

After a test preparation was orally administered to beagles of 30 to 42 months age where one group comprised 2 to 10 beagles, blood was taken and extracted and then concentration of the drug in plasma was measured by means of LC-MS/MS. The feeding condition was that beagles were placed in a cage with free access to water, fasted for 20 hours or more and a feed where 250 g of solid dog food and 50 g of chum were mixed was given. After 30 minutes from the feeding, the test preparation and 20 ml of water were administered. Taking of the blood was conducted in an amount of 1 mL from vein of brachial periodically using a syringe containing heparin. As to a stabilizer, EGTA (0.5 mg) and reduced glutathione (0.3 mg) were suspended in a mixture of 50% of N,N-dimethylacetamide and 50% of distilled water and previously placed in tubes for total blood and for plasma.
An extracting operation was as follows. Thus, firstly, 0.025 mL of 2N aqueous solution of hydrochloric acid was added to a tube. After that, 0.1 mL of internal standard substance (a solution of 100 ng/mL metformin in acetonitrile) was added to 0.1 mL of plasma prepared hereinabove and 0.3 mL of acetonitrile was added thereto to remove protein. After centrifugal separation, 0.005 mL of the resulting supernatant liquid was injected into LC-MS/MS. Measurement was conducted using LC-MS/MS under the following condition. A calibration curve was prepared by conducting the same operation as above for a preparation where internal standard substance (a solution of 100 ng/mL metformin in acetonitrile), levodopa and carbidopa were appropriately added to 0.1 mL of a blank plasma.

LC condition was as follows.
Apparatus: Agilent 1100
Column: Cadenza C18, 3 µm, 4.6 × 50 mm
Mobile phase: Aqueous solution of 10 mM ammonium acetate (pH 4.5) (A) / acetonitrile (B)

**[Table 2]**

| (minute(s)) | A (%) | B (%) |
|---|---|---|
| 0.0 | 90 | 10 |
| 3.0 | 90 | 10 |
| 4.0 | 10 | 90 |
| 7.0 | 10 | 90 |
| 7.1 | 90 | 10 |
| 12.0 | 90 | 10 |

Column temperature: 40°C
Flow rate: 0.5 mL/min
MS/MS conditions
Apparatus: API-4000
Ionizing method: ESI (positive) (Trubo Ion Spray)

**[Table 3]**

| Test Nos. | Preparation Administered | N (numbers of beagles) | Cmax (ng/ml) mean ± SD | Tmax (hr) mean ± SD | AUC (O-X) (ng×hr/ml) mean ± SD | MRT (hr) mean ± SD |
|---|---|---|---|---|---|---|
| 1 | Example 1 | 9 | 1615 ± 866 | 3.4 ± 1.8 | 10468 ± 2852 | 5.9 ± 1.3 |
| 2 | Example 2 | 2 | 1622 | 4.0 | 10118 | 4.9 |
| 3 | Example 3 | 3 | 1687 ± 433 | 6.8 ± 3.0 | 11122 ± 2072 | 5.9 ± 1.2 |
| 4 | Example 4 | 6 | 1088 ± 73 | 5.2 ± 2.7 | 8084 ± 705 | 7.1 ± 1.1 |
| 5 | Example 4 (Two tablets were administered at the same time) | 6 | 4025 ± 1111 | 5.0 ± 3.0 | 29661 ± 4960 | 6.6 ± 0.9 |
| 6 | Example 5 | 3 | 981 ± 120 | 6.0 ± 2.0 | 6617 ± 995 | 5.4 ± 1.2 |
| 7 | Example 6 | 3 | 984 ± 113 | 4.7 ± 3.1 | 6354 ± 662 | 6.1 ± 2.0 |
| 8 | Comp Ex 1 | 6 | 8116 ± 8073 | 0.8 ± 0.4 | 15453 ± 4822 | 2.3 ± 0.8 |
| 9 | Comp Ex 2 | 3 | 992 ± 272 | 8.0 ± 0.0 | 6816 ± 612 | 6.2 ± 0.2 |
| 10 | Sinemet CR | 10 | 3727 ± 1951 | 1.5 ± 1.1 | 10564 ± 3748 | 2.8 ± 0.5 |
| 11 | Sinemet CR (Additionally administered after 6 hours) | 8 | 6105 ± 1793 | 6.6 ± 3.9 | 30716 ± 7528 | 6.2 ± 0.9 |

Cmax is the maximum concentration in blood; Tmax is the time until reaching the maximum concentration in blood; AUC is the area under the curve for drug concentration in blood vs. time; and MRT is mean retention time.

Mean retention time is usually an index for the persistence of the concentration in blood and it means that, when the mean retention time is longer, function as a sustained release preparation is higher.

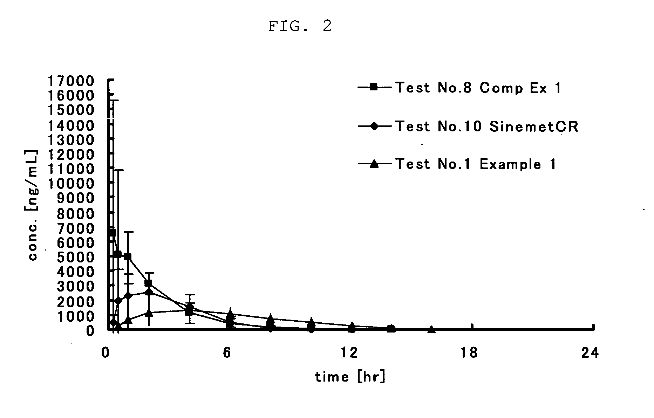

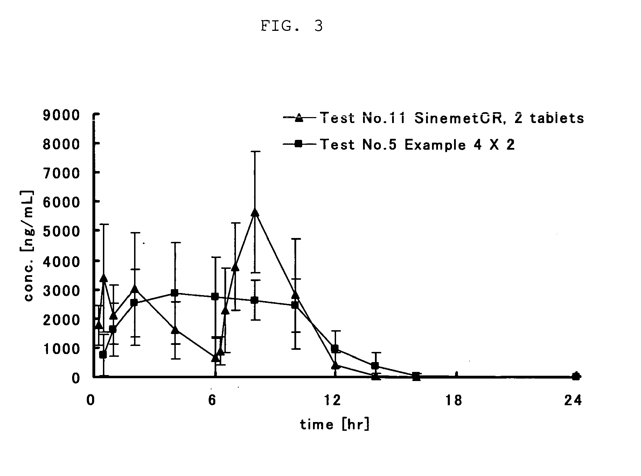

As mentioned above, the preparation according to the present invention maintains the concentration of levodopa in blood longer than Sinemet CR tablet does showing that levodopa is able to achieve a sustained release in the stomach (Fig. 2). In the group where Sinemet CR tablets were additionally administered after six hours, concentration in blood was unable to be kept constantly as compared with the group where the preparation of Example 4 was administered in two tablets at the same time (Fig. 3). Therefore, the preparation according to the present invention is a preparation which keeps the changes in concentration in blood constantly as compared with Sinemet CR tablets of a short-time releasing type and is a preparation which is able to overcome the problems in the levodopa preparations which are available at present. From the above result, the gastric retentive preparation according to the present invention is a preparation which is able to maximize the effect of levodopa.

### (dissolution test)

In accordance with a paddle method of a dissolution test method stipulated by the Japanese Pharmacopoeia, a test preparation was placed in a sinker, immersed in the JP first fluid (900 mL) and stirred at 37°C for 24 hours at a paddle speed of 100 rpm. The test solution was sampled periodically and the released rate of the drug was measured by an HPLC under the following condition.

Apparatus: Shimadzu Prominence
Column: Inertsil ODS-3, 5 µm, 4.6 × 150 mm
Column temperature: 25°C
Mobile phase: 20 mM potassium dihydrogen phosphate ((pH = 2.3) : acetonitrile (= 96:4)
Flow rate: 1 mL/min
Detector: UV of 280 nm

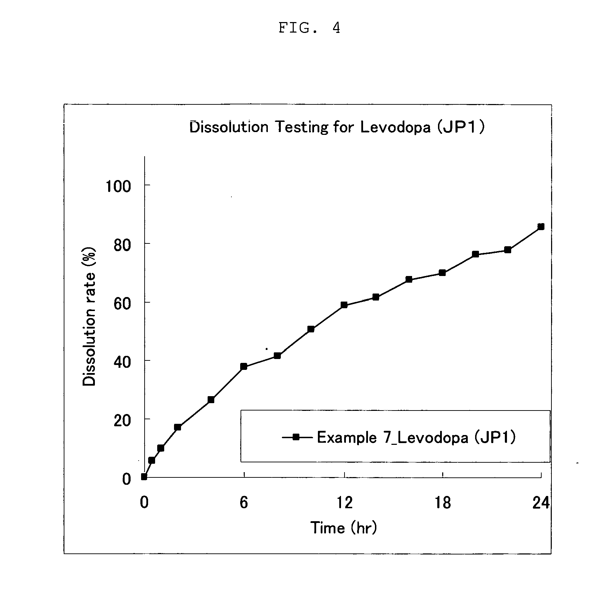

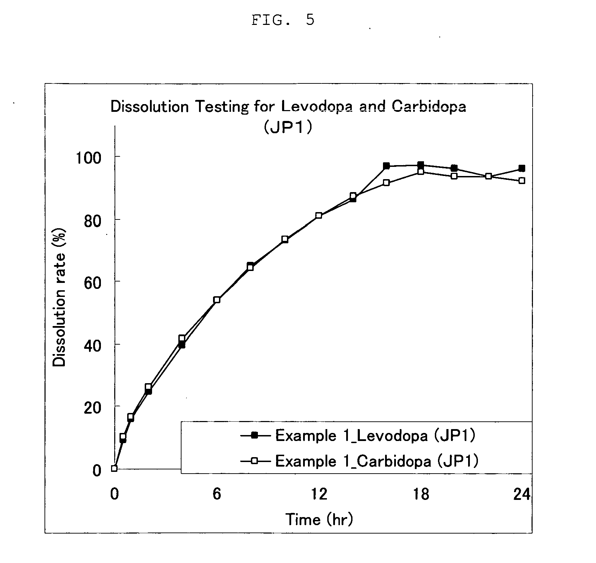

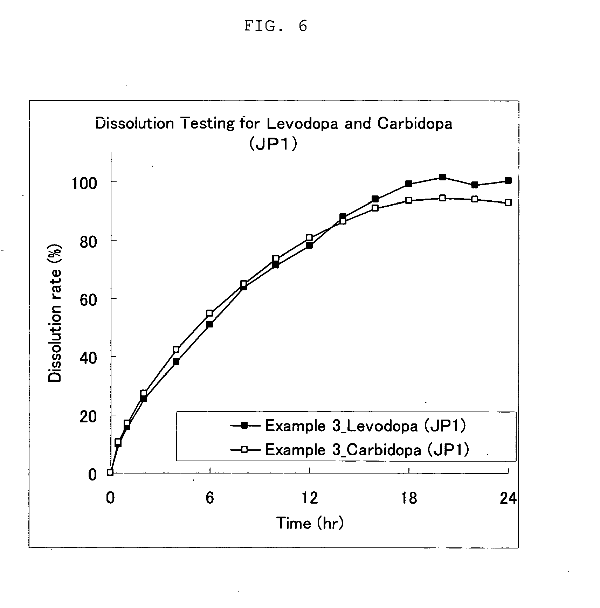

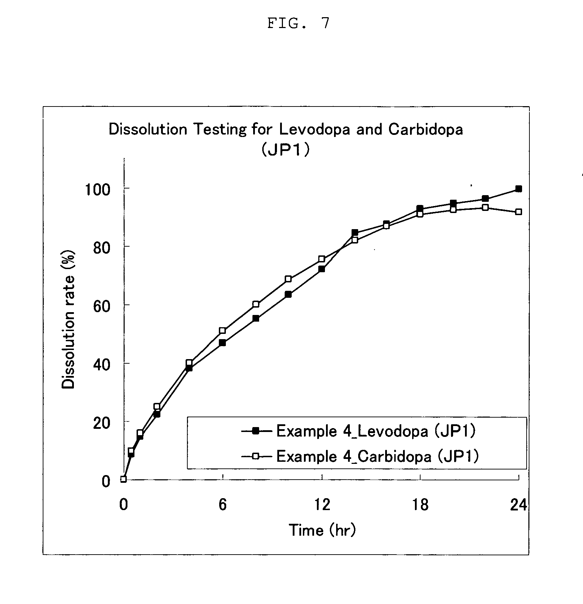

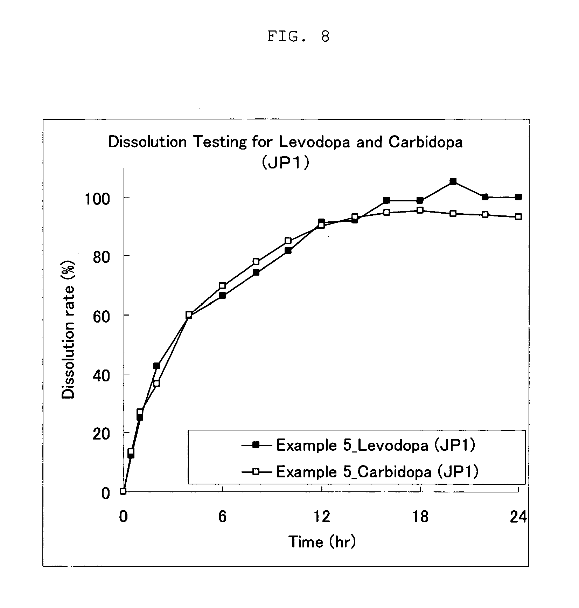

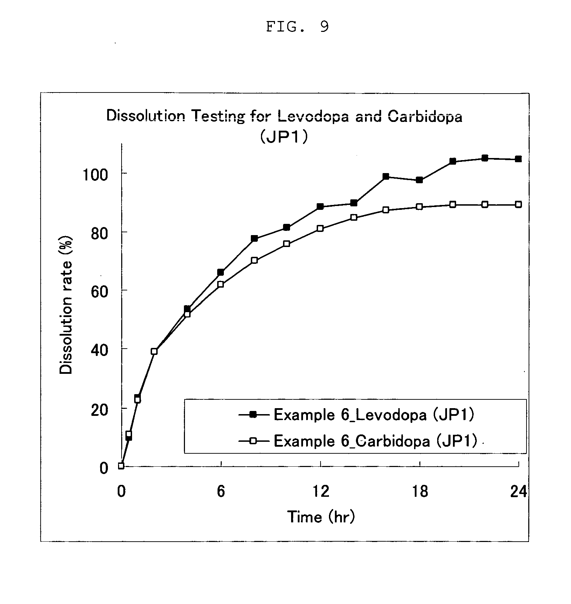

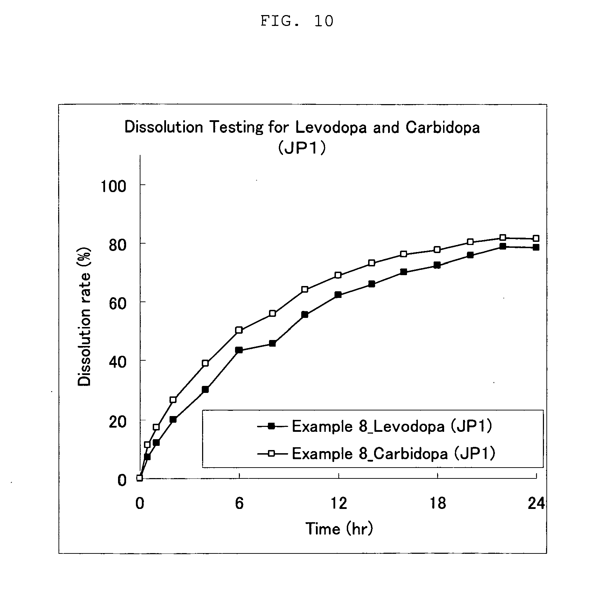

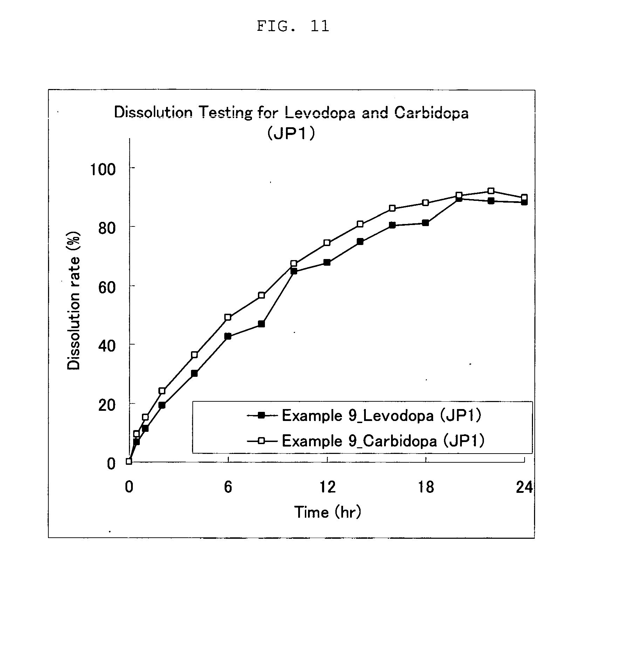

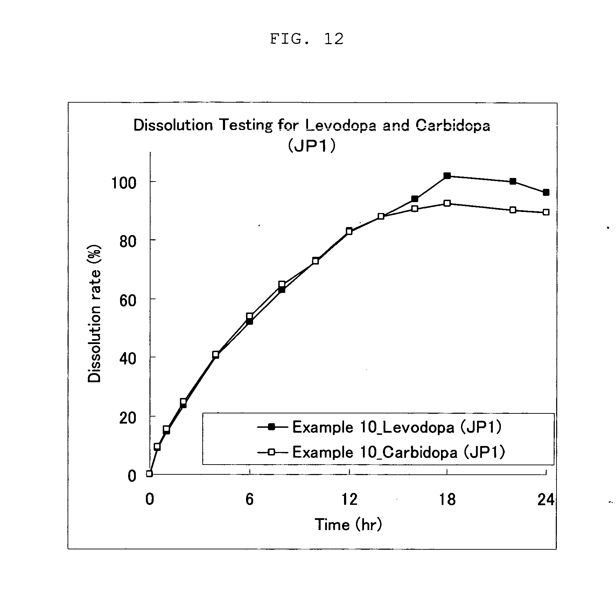

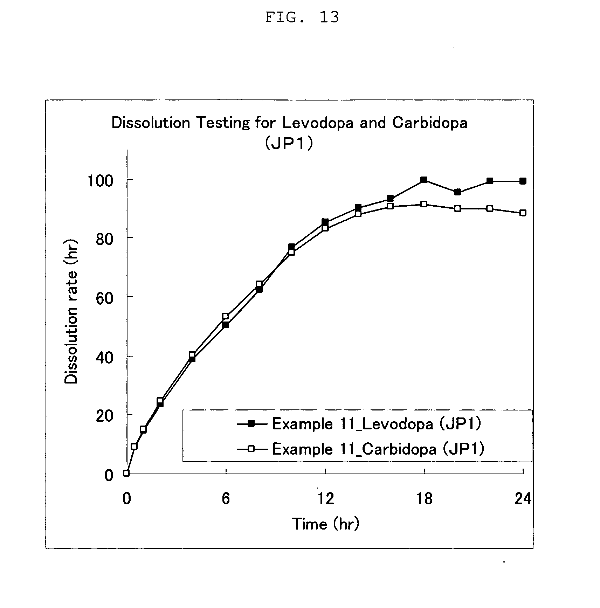

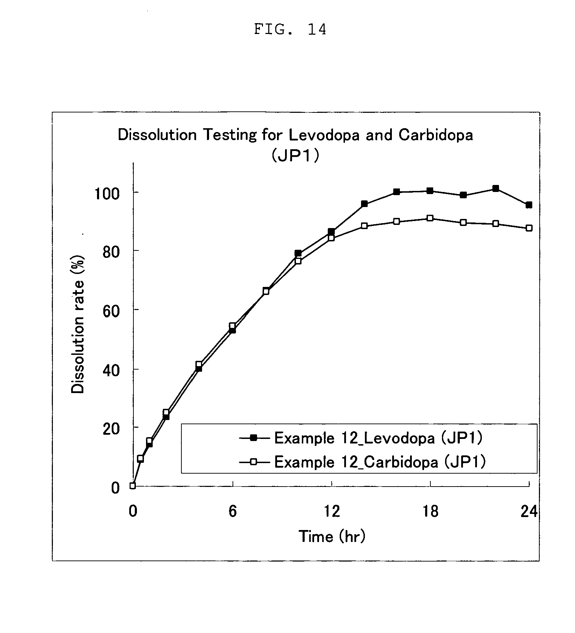

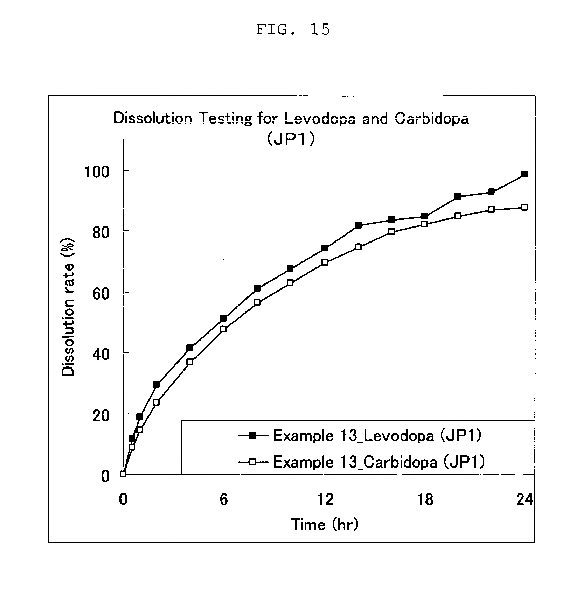

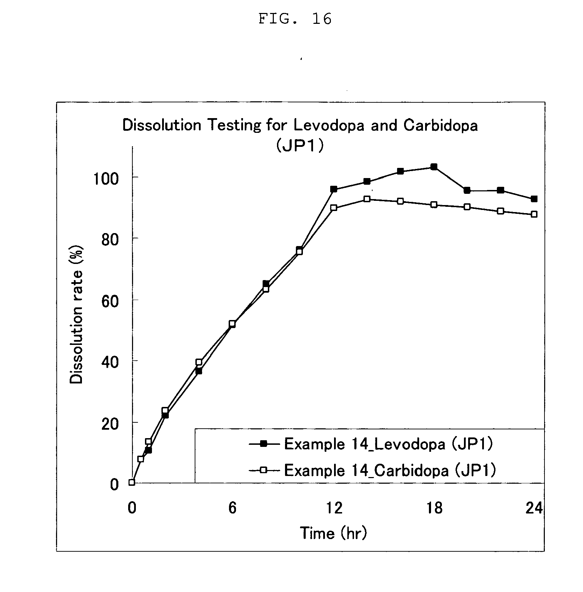

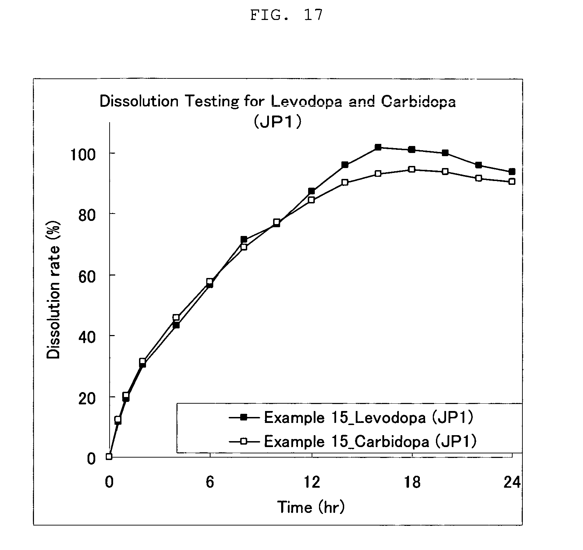

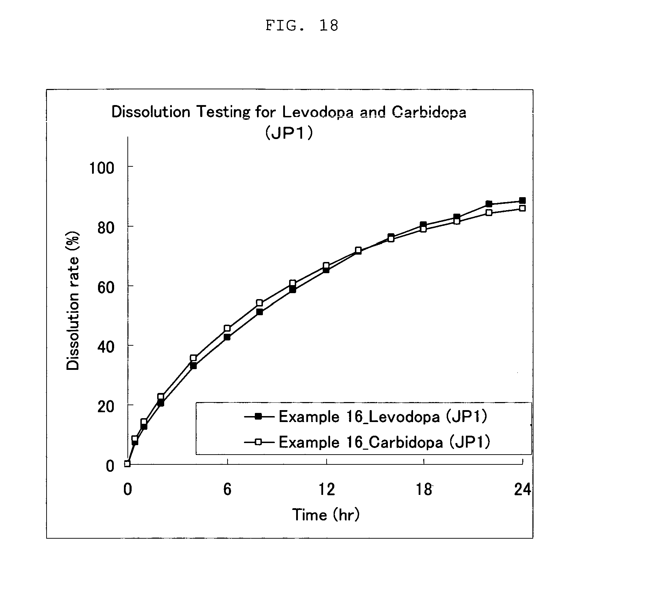

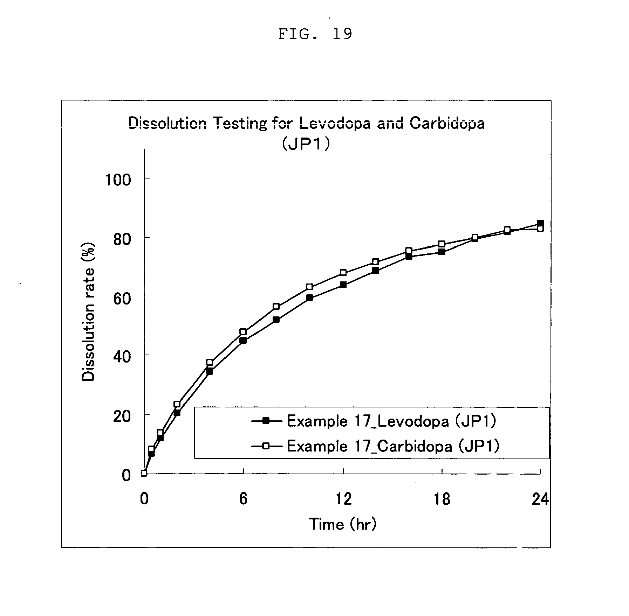

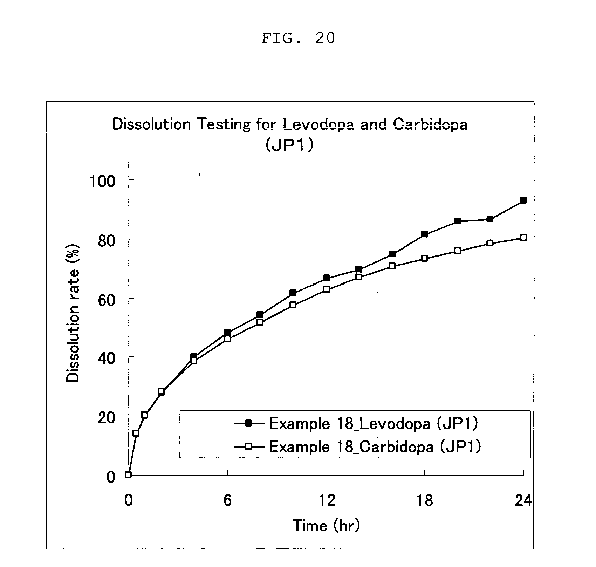

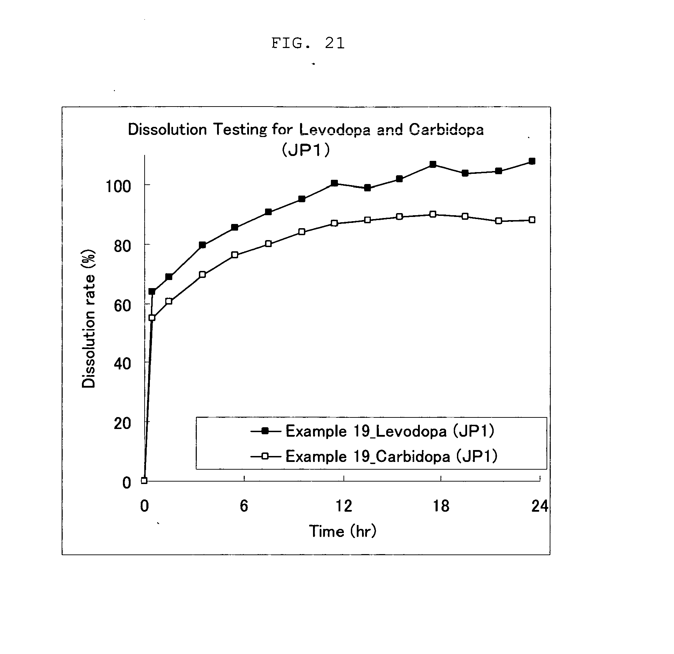

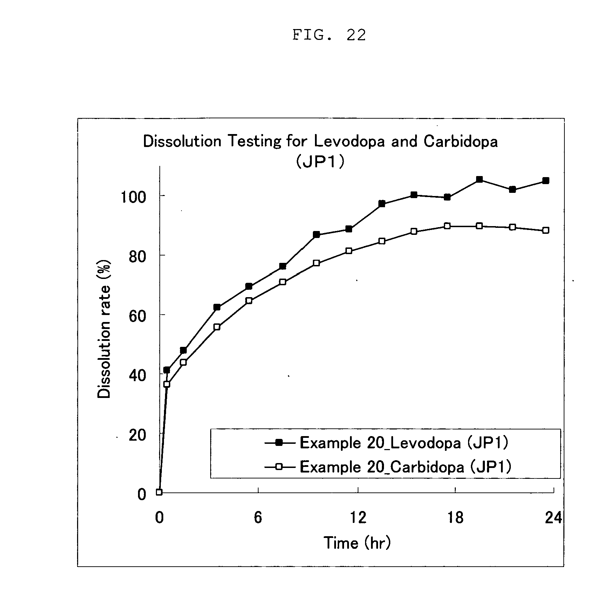

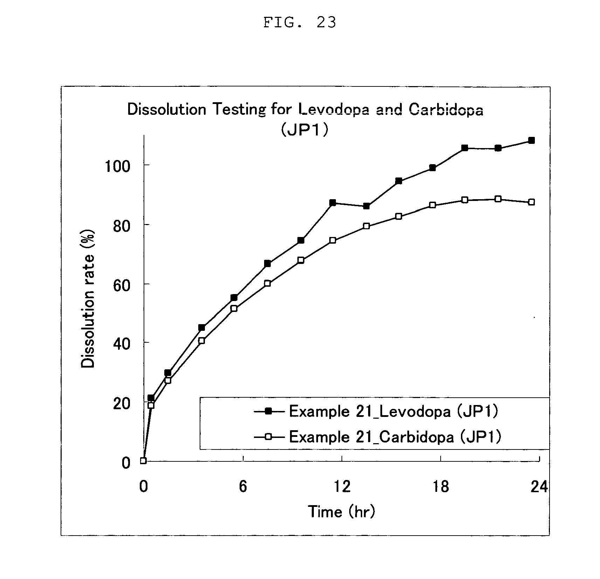

It is concluded from the result shown in Figs. 4 to 23 that the preparation according to the present invention is a preparation which persistently releases levodopa and/or carbidopa in an almost constant rate.

### [Industrial Applicability]

A gastric retentive preparation according to the present invention has a sufficient gastric residence time, has a size that enables easy ingestion and can disintegrate quickly after expelled from the stomach. The gastric retentive preparation is therefore useful as a sustained release preparation and is also a levodopa preparation which is easily prepared in industry.

## Claims

1. A gastric retentive preparation comprising a gastric resident layer and a drug releasing layer containing levodopa as a medicament, wherein the gastric resident layer includes a polymer ingredient that has higher solubility or swellability in a weakly acidic to weakly alkaline medium than in an acidic medium.

2. The preparation according to claim 1, wherein the polymer ingredient that has higher solubility or swellability in a weakly acidic to weakly alkaline medium than in an acidic medium is an enteric ingredient.

3. The preparation according to claim 2, wherein the enteric ingredient is selected from the group consisting of enteric cellulose derivatives, enteric acrylic acid copolymers, enteric polyvinyl derivatives and enteric maleic acid-vinyl copolymers.

4. The preparation according to claim 2 or 3, wherein the gastric resident layer further includes a water-soluble ingredient selected from the group consisting of hydroxyalkyl celluloses, polyvinyl polymers, polyethylene oxides, methyl cellulose, carboxymethyl celluloses, gelatin, polysaccharides, macrogols, sugars and sugar alcohols.

5. The preparation according to claim 4, wherein the rate of the enteric ingredient in the gastric resident layer is not less than 50% by weight of the gastric resident layer.

6. The preparation according to claim 1, wherein the polymer ingredient that has higher solubility or swellability in a weakly acidic to weakly alkaline medium than in an acidic medium is an acidic functional group-containing polymer ingredient selected from the group consisting of carboxymethyl celluloses, polyacrylic acids and polysaccharides having a carboxyl group or a sulfo group.

7. The preparation according to claim 6, wherein the gastric resident layer further includes a hydrophobic ingredient selected from the group consisting of cellulose derivatives, acrylic acid copolymers, vinyl acetate polymers, oils and fats, higher fatty acids, higher fatty acid esters, higher alcohols, hydrocarbons, polycaprolactone, polylactate, polyglycolate, and polyamides.

8. The preparation according to claim 7, wherein the rate of the hydrophobic ingredient to 1 part by weight of the acidic functional group-containing polymer ingredient is 1 to 40 parts by weight.

9. The preparation according to claim 7 or 8, wherein the gastric resident layer further includes a water-soluble ingredient selected from the group consisting of hydroxyalkyl celluloses, polyvinyl polymers, polyethylene oxides, methyl cellulose, carboxymethyl celluloses, gelatin, polysaccharides, macrogols, sugars and sugar alcohols.

10. The preparation according to claim 9, wherein the rate of the water-soluble ingredient in the gastric resident layer is not more than 60% by weight of the gastric resident layer.

11. The preparation according to claim 10, wherein the rate of the water-soluble ingredient in the gastric resident layer is not less than 5% by weight of the gastric resident layer.

12. The preparation according to any of claims 1 to 11, wherein the drug releasing layer includes hydroxyalkyl celluloses and/or polyethylene oxides.

13. The preparation according to any of claims 1 to 12, wherein a dopa decarboxylase inhibitor is further contained as a medicament.

14. The preparation according to any of claims 1 to 13, wherein a catechol-O-methyltransferase inhibitor is further contained as a medicament.

15. The preparation according to any of claims 1 to 14, wherein the rate of the gastric resident layer to the total weight of the preparation is 5 to 50% by weight.

16. The preparation according to any of claims 1 to 15, wherein the drug releasing layer contains a sustained release drug releasing layer and an immediate release drug releasing layer.
